# EUROPEAN PATENT APPLICATION

(11) **EP 3 480 711 A1**
(43) Date of publication of application: **08.05.2019**
(21) Application number: 17791532.9
(22) Date of filing: 05.04.2017
(51) Int. Cl.: G06F 17/30, G06Q 50/22

(54) **SEARCH SYSTEM, INFORMATION PROVIDING SYSTEM, CLIENT SIDE DEVICE, INFORMATION PROVIDING METHOD, INFORMATION PROVIDING PROGRAM, AND CLIENT SIDE PROGRAM**

(30) Priority: 29.06.2016 JP 2016128614
(71) Applicant: Reasonwhy Inc., Tokyo 105-0001 (JP)
(72) Inventor: SHIWAKU Tetsuo, Tokyo 105-0001 (JP)
(74) Representative: Pfenning, Meinig & Partner mbB
(86) International application number: PCT/JP2017/014240
(87) International publication number: WO 2018/003224

(57) **Abstract**

[Problem to be Solved]

To provide a retrieval system, an information providing system, a client-side device, a host-side device, an information providing method, an information providing program, a client-side program, and a host-side program that make it possible to improve retrieval performance of a host.

[Solution]

A retrieval system 1 is a retrieval system comprising a client-side device 10, a plurality of host-side devices 20, and an information providing devices 30. The client-side device 10 comprises a client-side communication unit, an acquisition unit for acquiring generation information, a retrieval condition information generation unit for generating retrieval condition information, and a display. Each of the plurality of host-side devices 20 comprises a host-side communication unit, a reply information generation unit for generating reply information, and a display. The information providing unit 30 comprises a communication unit, a specification unit for specifying desired host information from among host information stored in a host DB storing the retrieval condition information and the host information in association with each other, and a notification information generation unit for generating notification information.

## Description

### [Technical Field]

The present invention relates to a retrieval system, an information providing system, a client-side device, an information providing method, an information providing program, and a client-side program.

### [Background Art]

Conventionally, when a patient is urgently transported to a hospital by an ambulance, symptoms of the patient are diagnosed at the hospital of the destination of transport, and medical treatment is given to the patient. Herein, when medical treatment cannot be given to the patient due to insufficiency in equipment for medical devices, absence of the doctor who gives medical treatment, or the like in the hospital of the destination of transport, the patient is to be transported to another hospital. In this case, for example, when retrieval is performed until the above other hospital is found using a telephone, it takes too many times for the retrieval so that medical treatment to the patient delays, which is a risk in that the life of the patient becomes endangered.

To solve such a problem, a system has been proposed for retrieving a hospital on the basis of the accommodation state in the hospital and reserving the retrieved hospital (e.g., see Patent Literature 1).

### [Citation List]

### [Patent Literature]

Patent Literature 1: Japanese Patent Laid-Open No. 2005-000281

### [Summary of Invention]

### [Problems to be Solved by Invention]

However, in the above conventional technique, whether a doctor of the retrieved hospital is in a state capable of accepting the patient is not considered, so that even when the hospital is reserved, when the doctor who gives medical treatment is in surgery, it takes too much time until medical treatment is given to the patient, so that there is room for improvement in terms of retrieval performance.

The present invention has been conceived in light of the above-described problems, and an object thereof is to provide a retrieval system, an information providing system, a client-side device, an information providing method, an information providing program, and a client-side program that make it possible to improve retrieval performance of a host such as a doctor in a hospital.

### [Solution to Problem]

A retrieval system according to claim 1 is a retrieval system for retrieving a host that provides a service to a client, the retrieval system comprising a client-side device, a plurality of host-side devices, and an information providing system communicably connected to the client-side device and each of the plurality of host-side devices, wherein the client-side device comprises: client-side communication unit for communicating with the plurality of host-side devices or the information providing system; acquisition unit for acquiring generation information for generating retrieval condition information indicating a condition for retrieving the host; retrieval condition information generation unit for generating the retrieval condition information; client-side output unit for outputting various pieces of information related to the client-side device; and a medical care field table storing symptom information indicating a symptom of a patient and information indicating a medical care field in association with each other, each of the plurality of host-side devices comprises: host-side communication unit for communicating with the client-side device or the information providing system; reply information generation unit for generating reply information indicating a reply of whether the service can be provided; and host-side output unit for outputting various pieces of information related to the each of the host-side devices, and the information providing system comprises: communication unit for communicating with the client-side device or the plurality of host-side devices; host information storage unit for storing the retrieval condition information and host information for specifying the host in association with each other; specification unit for specifying the host information corresponding to the retrieval condition information from among the host information stored in the host information storage unit when the retrieval condition information is received by the communication unit; and notification information generation unit for generating notification information for giving a notice of being a host corresponding to the host information specified by the specification unit, and wherein the retrieval condition information generation unit of the client-side device generates the retrieval condition information based on the generation information when the generation information is acquired by the acquisition unit, the client-side communication unit of the client-side device transmits the retrieval condition information generated by the retrieval condition information generation unit to the information providing system, the specification unit of the information providing system specifies the host information corresponding to the retrieval condition information from among the host information stored in the host information storage unit when the retrieval condition information is received by the communication unit, the communication unit of the information providing system transmits the notification information generated by the notification information generation unit to one or more of the host-side devices corresponding to the host information specified by the specification unit, the host-side output unit of each of the plurality of host-side devices outputs the notification information when the notification information is received by the host-side communication unit, the reply information generation unit of each of the plurality of host-side devices generates the reply information on the notification information output by the host-side output unit and that indicates the reply specified by a predetermined method, the host-side communication unit of each of the plurality of host-side devices transmits the reply information generated by the reply information generation unit to the client-side device or the information providing system, the client-side output unit of the client-side device outputs the reply information when the reply information is received by the client-side communication unit, the service is a medical service, the retrieval condition information includes medical care field information indicating a medical care field of the doctor related to the symptom of the patient and attendance state information indicating an attendance state of the doctor, or location information indicating a location of the doctor related to location of the patient, the generation information includes medical care field generation information for generating the medical care field information, the medical care field generation information indicating the symptom of the patient, and attendance state generation information for generating the attendance state information, the attendance state generation information indicating the attendance state of the doctor, or location generation information for generating the location information, the location generation information indicating an area in a predetermined distance range from a current location of the patient or a client assistant, and the retrieval condition information generation unit of the client-side device specifies, when the medical care field generation information is acquired by the acquisition unit, information indicating the medical care field corresponding to the medical care field generation information from among information indicating the medical care field stored in the medical care field table, and generates the specified information as the medical care field information, generates, when the attendance state generation information is acquired by the acquisition unit, the attendance state generation information as the attendance state information, and extracts, when the location generation information is acquired by the acquisition unit, the location information corresponding to coordinates in the area indicated by the location generation information from among the location information acquired by the information providing system or the host-side devices, and generates the extracted information as the location information.

A retrieval system according to claim 2 is a retrieval system for retrieving a host that provides a service to a client, the retrieval system comprising a client-side device, a plurality of host-side devices, and an information providing system communicably connected to the client-side device and each of the plurality of host-side devices, wherein the client-side device comprises: client-side communication unit for communicating with the plurality of host-side devices or the information providing system; acquisition unit for acquiring generation information for generating retrieval condition information indicating a condition for retrieving the host; retrieval condition information generation unit for generating the retrieval condition information; and client-side output unit for outputting various pieces of information related to the client-side device, each of the plurality of host-side devices comprises: host-side communication unit for communicating with the client-side device or the information providing system; reply information generation unit for generating reply information indicating a reply of whether the service can be provided; and host-side output unit for outputting various pieces of information related to the each of the host-side devices, and the information providing system comprises: communication unit for communicating with the client-side device or the plurality of host-side devices; host information storage unit for storing the retrieval condition information and host information for specifying the host in association with each other; specification unit for specifying host information corresponding to the retrieval condition information from among the host information stored in the host information storage unit when the retrieval condition information is received by the communication unit, and notification information generation unit for generating notification information for giving a notice of being a host corresponding to the host information specified by the specification unit, and wherein the retrieval condition information generation unit of the client-side device generates the retrieval condition information based on the generation information when the generation information is acquired by the acquisition unit, the client-side communication unit of the client-side device transmits the retrieval condition information generated by the retrieval condition information generation unit to the information providing system, the specification unit of the information providing system specifies the host information corresponding to the retrieval condition information from among the host information stored in the host information storage unit when the retrieval condition information is received by the communication unit, the communication unit of the information providing system transmits the notification information generated by the notification information generation unit to one or more of the host-side devices corresponding to the host information specified by the specification unit, the host-side output unit of each of the plurality of host-side devices outputs the notification information when the notification information is received by the host-side communication unit, the reply information generation unit of each of the plurality of host-side devices generates the reply information on the notification information output by the host-side output unit and that indicates the reply specified by a predetermined method, the host-side communication unit of each of the plurality of host-side devices transmits the reply information generated by the reply information generation unit to the client-side device or the information providing system, the client-side output unit of the client-side device outputs the reply information when the reply information is received by the client-side communication unit, the service is a medical service, the retrieval condition information includes attendance state information indicating an attendance state of the doctor, or location information indicating a location of the doctor related to a location of the patient, the generation information includes attendance state generation information for generating the attendance state information, the attendance state generation information indicating the attendance state of the doctor, or location generation information for generating the location information, the location generation information indicating an area in a predetermined distance range from a current location of the patient or a client assistant, and the retrieval condition information generation unit of the client-side device generates, when the attendance state generation information is acquired by the acquisition unit, the attendance state generation information as the attendance state information, and extracts, when the location generation information is acquired by the acquisition unit, the location information corresponding to coordinates in the area indicated by the location generation information from among the location information acquired by the information providing system or the host-side devices, and generates the extracted information as the location information.

An information providing system according to claim 3 is an information providing system communicably connected to a client-side device and each of a plurality of host-side devices, the information providing system comprising: communication unit for communicating with the client-side device or the plurality of host-side devices; host information storage unit for storing retrieval condition information indicating a condition for retrieving a host that provides a service to a client, and host information for specifying the host in association with each other; specification unit for specifying host information corresponding to the retrieval condition information from among the host information stored in the host information storage unit when the retrieval condition information is received by the communication unit; and notification information generation unit for generating notification information for giving a notice of being a host corresponding to the host information specified by the specification unit, wherein the specification unit specifies the host information corresponding to the retrieval condition information from among the host information stored in the host information storage unit when by the communication unit receives the retrieval condition information from the client-side device, the communication unit transmits the notification information generated by the notification information generation unit to one or more of the host-side devices corresponding to the host information specified by the specification unit, the service is a medical service, and the retrieval condition information includes location information indicating a location of the doctor related to a location of the patient.

In accordance with the invention according to claim 3, the information providing system according to claim 4 comprising update unit for updating, when doctor current location information indicating a current location of the doctor and medical facility location information indicating a location of a medical facility to which the doctor belongs are acquired by a predetermined method, the attendance state information among the retrieval condition information stored in the host information storage unit based on the doctor current location information and the medical facility location information.

In accordance with the invention according to claim 3 or claim 4, the information providing system according to claim 5, wherein the communication unit of the information providing system transmits, when the communication unit receives confirmation information indicating that the host that provides the service has confirmed from the client-side device, the confirmation information to one or more of the host-side devices other than one of the host-side devices corresponding to the confirmation information from among some of the host-side devices corresponding to the host information specified by the specification unit.

A client-side device according to claim 6 is a client-side device communicably connected to an information providing device and each of a plurality of host-side devices, the client-side device comprising: client-side communication unit for communicating with the plurality of host-side devices or the information providing system; acquisition unit for acquiring generation information for generating retrieval condition information indicating a condition for retrieving a host; retrieval condition information generation unit for generating the retrieval condition information; client-side output unit for outputting various pieces of information related to the client-side device; and a medical care field table storing symptom information indicating a symptom of a patient and information indicating a medical care field in association with each other, wherein the retrieval condition information generation unit generates the retrieval condition information based on the generation information when the generation information is acquired by the acquisition unit, the client-side communication unit transmits the retrieval condition information generated by the retrieval condition information generation unit to the information providing system, the client-side output unit outputs, when the client-side communication unit receives reply information indicating a reply of whether a service can be provided to a client, the reply information, the service is a medical service, the retrieval condition information includes medical care field information indicating a medical care field of the doctor related to the symptom of the patient and attendance state information indicating an attendance state of the doctor, or location information indicating a location of the doctor related to location of the patient, the generation information includes medical care field generation information for generating the medical care field information, the medical care field generation information indicating the symptom of the patient, and attendance state generation information for generating the attendance state information, the attendance state generation information indicating the attendance state of the doctor, or location generation information for generating the location information, the location generation information indicating an area in a predetermined distance range from a current location of the patient or a client assistant, and the retrieval condition information generation unit specifies, when the medical care field generation information is acquired by the acquisition unit, information indicating the medical care field corresponding to the medical care field generation information from among information indicating the medical care field stored in the medical care field table, and generates the specified information as the medical care field information, and extracts, when the location generation information is acquired by the acquisition unit, the location information corresponding to coordinates in the area indicated by the location generation information from among the location information acquired by the information providing system or the host-side devices, and generates the extracted information as the location information.

A client-side device according to claim 7 is a client-side device communicably connected to an information providing device and each of a plurality of host-side devices, the client-side device comprising: client-side communication unit for communicating with the plurality of host-side devices or the information providing system; acquisition unit for acquiring generation information for generating retrieval condition information indicating a condition for retrieving a host; retrieval condition information generation unit for generating the retrieval condition information; client-side output unit for outputting various pieces of information related to the client-side device; and a medical care field table storing symptom information indicating symptom of a patient and information indicating a medical care field in association with each other, wherein the retrieval condition information generation unit generates the retrieval condition information based on the generation information when the generation information is acquired by the acquisition unit, the client-side communication unit transmits the retrieval condition information generated by the retrieval condition information generation unit to the information providing system, the client-side output unit outputs, when the client-side communication unit receives reply information indicating a reply of whether a service can be provided to a client, the reply information, the service is a medical service, the retrieval condition information includes attendance state information indicating an attendance state of a doctor, or location information indicating a location of the doctor related to a location of the patient, the generation information includes attendance state generation information for generating the attendance state information, the attendance state generation information indicating the attendance state of the doctor, or location generation information for generating the location information, the location generation information indicating an area in a predetermined distance range from a current location of the patient or a client assistant, and the retrieval condition information generation unit of the client-side device generates, when the attendance state generation information is acquired by the acquisition unit, the attendance state generation information as the attendance state information, and extracts, when the location generation information is acquired by the acquisition unit, the location information corresponding to coordinates in the area indicated by the location generation information from among the location information acquired by the information providing system or the host-side devices, and generates the extracted information as the location information.

An information providing method according to claim 8 is an information providing method performed in an information providing system communicably connected to a client-side device and each of a plurality of host-side devices, the information providing method comprising: a communication step for communication unit communicating with the client-side device or the plurality of host-side devices; a specification step for specification unit specifying, when retrieval condition information indicating a condition for retrieving a host that provides a service to a client is received by the communication unit, host information corresponding to the received retrieval condition information from among host information for specifying the host stored in host information storage unit with the retrieval condition information in association to each other; and a notification information generation step for notification information generation unit generating notification information for giving a notice of being a host corresponding to the host information specified in the specification step; wherein in the specification step, the host information corresponding to the retrieval condition information is specified from among the host information stored in the host information storage unit when the retrieval condition information is received from the client-side device in the communication step, in the communication step, the notification information generated in the notification information generation step is transmitted to one or more of the host-side devices corresponding to the host information specified in the specification step, the service is a medical service, and the retrieval condition information includes location information indicating a location of the doctor related to a location of the patient.

An information providing program according to claim 9 is an information providing program for being executed by an information providing system communicably connected to a client-side device and each of a plurality of host-side devices, the information providing program causing a computer to function as: communication unit for communicating with the client-side device or the plurality of host-side devices; host information storage unit for storing retrieval condition information indicating a condition for retrieving a host that provides a service to a client and host information for specifying the host in association with each other; specification unit for specifying host information corresponding to the retrieval condition information from among the host information stored in the host information storage unit when the retrieval condition information is received by the communication unit; and notification information generation unit for generating notification information for giving a notice of being a host corresponding to the host information specified by the specification unit, wherein the specification unit specifies the host information corresponding to the retrieval condition information from among the host information stored in the host information storage unit when the communication unit receives the retrieval condition information from the client-side device, the communication unit transmits the notification information generated by the notification information generation unit to one or more of the host-side devices corresponding to the host information specified by the specification unit, the service is a medical service, and the retrieval condition information includes location information indicating a location of the doctor related to a location of the patient.

A client-side program according to claim 10 is a client-side program for being executed by a client-side device communicably connected to an information providing system and each of a plurality of host side devices, the client-side program causing a computer to function as: client-side communication unit for communicating with the plurality of host-side devices or the information providing system; acquisition unit for acquiring generation information for generating retrieval condition information indicating a condition for retrieving a host; retrieval condition information generation unit for generating the retrieval condition information; client-side output unit for outputting various pieces of information related to the client-side device; and a medical care field table storing symptom information indicating a symptom of a patient and information indicating a medical care field in association with each other, wherein the retrieval condition information generation unit generates the retrieval condition information based on the generation information when the generation information is acquired by the acquisition unit, the client-side communication unit transmits the retrieval condition information generated by the retrieval condition information generation unit to the information providing system, the client-side output unit outputs, when the client-side communication unit receives reply information indicating a reply of whether a service can be provided to a client, the reply information, the service is a medical service, the retrieval condition information includes medical care field information indicating a medical care field of the doctor related to the symptom of the patient, and attendance state information indicating an attendance state of a doctor, or location information indicating a location of the doctor related to location of the patient, the generation information includes medical care field generation information for generating the medical care field information, the medical care field generation information indicating the symptom of the patient, and attendance state generation information for generating the attendance state information, the attendance state generation information indicating the attendance state of the doctor, or location generation information for generating the location information, the location generation information indicating an area in a predetermined distance range from a current location of the patient or a client assistant, and the retrieval condition information generation unit specifies, when the medical care field generation information is acquired by the acquisition unit, information indicating the medical care field corresponding to the medical care field generation information from among information indicating the medical care field stored in the medical care field table, and generates the specified information as the medical care field information, generates, when the attendance state generation information is acquired by the acquisition unit, the attendance state generation information as the attendance state information, and extracts, when the location generation information is acquired by the acquisition unit, the location information corresponding to coordinates in the area indicated by the location generation information from among the location information acquired by the information providing system or the host-side devices, and generates the extracted information as the location information.

A client-side program according to claim 11 is a client-side program for being executed by a client-side device communicably connected to an information providing system and each of a plurality of host side devices, the client-side program causing a computer to function as: client-side communication unit for communicating with the plurality of host-side devices or the information providing system; acquisition unit for acquiring generation information for generating retrieval condition information indicating a condition for retrieving a host; retrieval condition information generation unit for generating the retrieval condition information; client-side output unit for outputting various pieces of information related to the client-side device; and a medical care field table storing symptom information indicating symptom of a patient and information indicating a medical care field in association with each other, wherein the retrieval condition information generation unit generates the retrieval condition information based on the generation information when the generation information is acquired by the acquisition unit, the client-side communication unit transmits the retrieval condition information generated by the retrieval condition information generation unit to the information providing system, the client-side output unit outputs, when the client-side communication unit receives reply information indicating a reply of whether a service can be provided to a client, the reply information, the service is a medical service, the retrieval condition information includes attendance state information indicating an attendance state of a doctor, or location information indicating a location of the doctor related to a location of the patient, the generation information includes attendance state generation information for generating the attendance state information, the attendance state generation information indicating the attendance state of the doctor, or location generation information for generating the location information, the location generation information indicating an area in a predetermined distance range from a current location of the patient or a client assistant, and the retrieval condition information generation unit of the client-side device generates, when the attendance state generation information is acquired by the acquisition unit, the attendance state generation information as the attendance state information, and extracts, when the location generation information is acquired by the acquisition unit, the location information corresponding to coordinates in the area indicated by the location generation information from among the location information acquired by the information providing system or the host-side devices, and generates the extracted information as the location information.

### [Effects of inventions]

According to the retrieval system of claim 1, the information providing system of claim 3, the client-side device of claim 6, the information providing method of claim 8, the information providing program of claim 9, and the client-side program of claim 10, the client-side communication unit of the client-side device transmits the retrieval condition information generated by the retrieval condition information generation unit to the information providing device, the specification unit of the information providing device specifies the host information corresponding to the retrieval condition information from among the host information stored in the host information storing unit when the retrieval condition information is received by the communication unit, and the communication unit of the information providing device transmits the notification information generated by the notification information generation unit to the host-side devices corresponding to the host information specified by the specification unit, so that the notification information can be transmitted to all the host-side devices that match the retrieval condition information, making it possible to quickly and efficiently offer the notification information to the hosts of the host-side devices. Furthermore, the host-side communication unit of each of the plurality of host-side devices transmits the reply information generated by the reply information generation unit to the client-side device, and the output device of the client-side device outputs the reply information when the client-side communication unit receives the reply information, so that the client-side device can offer at least one piece of the reply information received from the host-side devices that match the retrieval condition information to the client, making it possible to determine the host who provides medical service from among the hosts corresponding to the reply information. This makes it possible to quickly and effectively retrieve the host who matches the needs of the client as compared with a conventional technique, so that the retrieval performance can be improved.

Furthermore, the service is medical service, and the retrieval condition information includes the attendance state information indicating an attendance state of a doctor, the medical care field information indicating a medical care field of a doctor related to a symptom of a patient, or the location information indicating a location of a doctor related to a location of a patient, so that the host that matches the needs of the client who receives medical service can be retrieved even more efficiently, making it possible to further improve the retrieval performance of the host.

Furthermore, the retrieval condition information includes attendance state information indicating an attendance state of the doctor, or location information indicating a location of the doctor related to a location of the patient, so that the host that matches the needs of the client who receives medical service can be retrieved even more efficiently, making it possible to further improve the retrieval performance of the host.

According to the retrieval system of claim 2, the client-side device of claim 7, and the client-side program of claim 11, the service is medical service, and the retrieval condition information includes the attendance state information indicating an attendance state of a doctor, or the location information indicating a location of a doctor related to a location of a patient, so that the host that matches the needs of the client who receives medical service can be retrieved even more efficiently, making it possible to further improve the retrieval performance of the host.

According to the information providing device of claim 4, the information providing device is equipped with the update unit that updates, when the doctor current location information and the medical facility location information are acquired by a predetermined method, the attendance state information among the retrieval condition information stored in the host information storing unit on the basis of the doctor current location information and the medical facility location information, so that the attendance state information stored in the host information storing unit can be updated without receiving a notification indicating attendance state from the host, making it possible to eliminate a trouble of an operation related to the update by the host.

According to the information providing device of claim 5, the communication unit of the information providing device transmits, when the confirmation information is received by the communication unit from the client-side device, the confirmation information to the host-side devices other than the host-side device corresponding to the confirmation information among the host-side devices corresponding to the host information specified by the specification unit, so that the confirmation information can be offered to the other host who do not provide service to the client among the hosts of the host-side devices specified by the specification unit, enabling the other host to afterward confirm that service has provided to the client by a host other than he/she.

### [Brief Description of Drawings]

Fig. 1 is a diagram conceptually illustrating a retrieval system according to an embodiment of the invention.
Fig. 2 is a block diagram illustrating an electrical configuration of a client-side device.
Fig. 3 is a block diagram illustrating an electrical configuration of a host-side device.
Fig. 4 is a block diagram illustrating an electrical configuration of an information providing device.
Fig. 5 is a diagram exemplifying information stored in a host DB.
Fig. 6 is a diagram exemplifying information stored in a medical facility DB.
Fig. 7 is a flowchart of retrieval processing related to the client-side device according to the embodiment.
Fig. 8 is a flowchart of retrieval processing related to the information providing device according to the embodiment.
Fig. 9 is a flowchart of retrieval processing related to the host-side device according to the embodiment.
Fig. 10 is a flowchart of update processing related to the information providing device according to the embodiment.

### [Detailed Description of Embodiment]

Hereinafter, an embodiment of a retrieval system, an information providing system, a client-side device, an information providing method, an information providing program, and a client-side program according to the invention will be described in detail with reference to the accompanying drawings.

### [Basic Concept of Embodiment]

First, a basic concept of the embodiment will be described.

The retrieval system according to the invention schematically is a system for retrieving a host that provides a service to a client. Herein, the "client" denotes a person who requests a service to a host and receives the service. Furthermore, the "host" denotes a person who receives the request of the service from a client and provides the service. Furthermore, the "service" denotes labor that is received by the client from the host, and is a concept including, for example, a medical service, a consulting service and the like. Furthermore, equipment that functions as the retrieval system is equipment for retrieving the host, and includes, for example, a plurality of pieces of equipment connected by wire or wirelessly, and specifically includes a client-side device, a host-side device, and an information providing device. Among the devices, the "client-side device" is a device owned by a client or a client-side assistant who retrieves a host instead of a client, and is a concept including, for example, a mobile terminal (e.g., a smart phone or a tablet terminal), a stationary personal computer or the like. Furthermore, the "host-side device" is a device owned by a host or a host-side assistant who assists business of a host, and is a concept including, for example, a mobile terminal (e.g., a smart phone or a tablet terminal), a stationary personal computer or the like. Furthermore, the "information providing device" is a device provided at a base station (omitted in the drawings) or the like, and is a concept including, for example, a stationary server. Hereinafter, in the embodiment, a case will be described in which service is medical service and although a patient (client) is urgently transported to a first medical facility, a doctor who gives medical treatment cannot provide a medical service (specifically, medical care service) because the doctor is in a peroperative period or the like, so that a doctor belonging to a second medical facility different from the first medical facility and capable of providing medical service to the patient (host; referred to as "second doctor" hereinafter) is retrieved. Furthermore, the client-side device shall be a device owned by a doctor belonging to the first medical facility (client-side assistant: referred to as "first doctor" hereinafter), and description will be made on the assumption that the first doctor retrieves a second doctor using the client-side device instead of the patient. Furthermore, the description will be made on the assumption that the host-side device is owned by a second doctor.

### (Specific Content of the Embodiment)

Next, specific contents of the embodiment will be described.

### (Configuration)

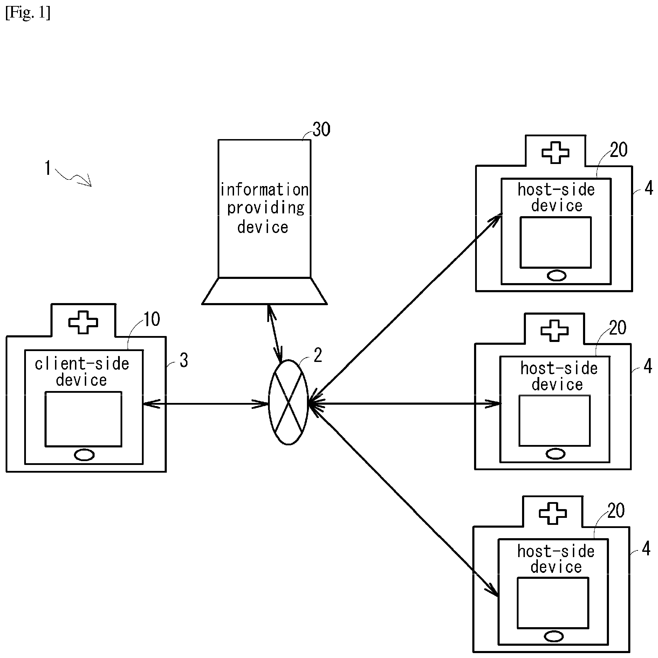

First, a configuration of the retrieval system according to an embodiment will be described. Fig. 1 is a diagram conceptually illustrating the retrieval system according to an embodiment of the invention. As illustrated in Fig. 1, the retrieval system 1 comprises a client-side device 10, a plurality of host-side devices 20, and an information providing devices 30, and the client-side device 10, the plurality of host-side devices 20, and the information providing devices 30 are mutually communicably connected via a network 2. Note that the plurality of host-side devices 20 can be configured in the same way, so that in the following description, the configuration of one of the host-side devices 20 will be described, and description of the configuration of the other host-side devices 20 will be omitted.

### (Configuration - client-side device)

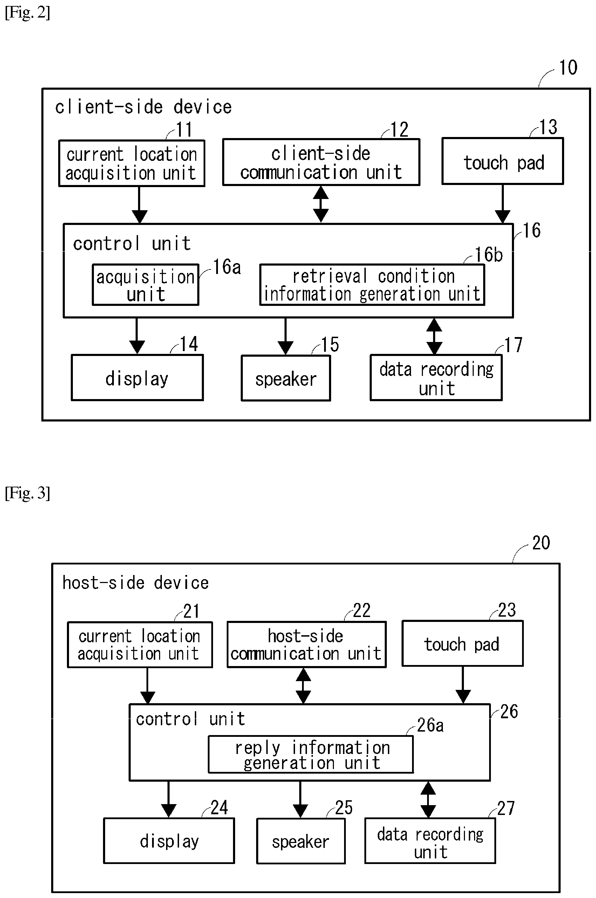

Next, a configuration of the client-side device 10 will be described. Fig. 2 is a block diagram illustrating an electrical configuration of the client-side device 10. The client-side device 10 is a device that transmits retrieval condition information described below, and as illustrated in Fig. 2, is configured to include a current location acquisition unit 11, a client-side communication unit 12, a touch pad 13, a display 14, a speaker 15, a control unit 16, and a data recording unit 17. Note that the client-side device 10 can be configured by, for example, a known mobile terminal or the like owned by the first doctor belonging to a first medical facility 3, so that its detailed description will be omitted.

### (Configuration - client-side device - current location acquisition unit)

The current location acquisition unit 11 is current location acquisition unit for detecting a current location of the patient or the first doctor on the basis of a GPS signal received from a satellite. The current location acquisition unit 11 is configured by using, for example, a GPS receiver, and detects the current location (coordinates), orientation, etc. of the patient (or the first doctor) by a known method (note that, the same applies to a following current location acquisition unit 21 of the host-side device 20).

### (Configuration - client-side device - client-side communication unit)

The client-side communication unit 12 is client-side communication unit for communicating with the plurality of the host-side device 20 or the information providing device 30, and for example, is configured by using known communication unit that performs communication by using a wired communication network or a wireless communication network (note that, the same applies to a host-side communication unit 22 of the host-side device 20 and a communication unit 31 of the communication providing device 30 described below).

### (Configuration - client-side device - touch pad)

The touch pad 13 is operation unit for receiving various operation inputs from the first doctor by, for example, being pressed by a finger of the first doctor. The specific configuration of this touch pad 13 is optional, and for example a known one provided with operation position detection unit of a resistive film type, a capacitive type or the like can be used (note that, the same applies to a touch pad 23 of the host-side device 20 described below).

### (Configuration - client-side device - display)

The display 14 is display unit for displaying various types of images on the basis of control of the control unit 16. The specific configuration of this display 14 is optional, and for example, a flat panel display such as a known liquid crystal display or an organic EL display can be used (note that, the same applies to a display 24 of the host-side device 20 described below). Note that, the touch pad 13 and the display 14 may be integrally formed as the touch pad 13.

### (Configuration - client-side device - speaker)

The speaker 15 is output unit for outputting various types of sounds on the basis of control of the control unit 16. A specific mode of the sound output from the speaker 15 is optional, and synthesized sound generated as necessary or sound recorded in advance can be output (note that, the same applies to a speaker 25 of the host-side device 20 described below). Note that the "display 14" and the "speaker 15" correspond to "client-side output unit" in CLAIMS.

### (Configuration - client-side device - control unit)

The control unit 15 is control unit for controlling the client-side device 10 and specifically, is a computer configured to comprise a CPU, various programs interpreted/executed on the CPU (including a basic control program such as OS and an application program activated on the OS and realizing a specific function), and an internal memory such as a RAM for storing the programs and various types of data. Particularly, a client-side program according to the embodiment is installed in the client-side device 10 through an arbitrary recording medium or via the network 2 so as to substantively configure each part of the control unit 16.

As illustrated in Fig. 2, the control unit 16 also comprises an acquisition unit 16a and a retrieval condition information generation unit 16b in terms of a functional concept. The acquisition unit 16a is acquisition unit for acquiring generation information described below. The retrieval condition information generation unit 16b is retrieval condition information generation unit for generating retrieval condition information described below. Note that the detail of the processing executed by the control unit 16 will be described below.

### (Configuration - client-side device - data recording unit)

The data recording unit 17 is recording unit for recording a program necessary to operate the client-side device 10 and various pieces of data (e.g., map information), and is configured by using, for example, a hard disc as an external recording device (omitted in the drawing). However, any other recording media including a magnetic recording medium such as a magnetic disc, an optical recording medium such as a DVD and a Blu-ray Disc, and an electrical recording medium such as a Flash ROM, a USB memory, and an SD card can be used instead of the hard disc or with the hard disc (note that, the same shall apply to a data recording unit 27 of the host-side device 20 described below, and a data recording unit 33 of the information providing device 30 described below).

### (Configuration - host-side device)

Next, a configuration of the host-side device 20 will be described. Fig. 3 is a block diagram illustrating an electrical configuration of the host-side device 20. The host-side device 20 is a device that transmits reply information described below, and as illustrated in Fig. 3, is configured to comprise a current location acquisition unit 21, a host-side communication unit 22, a touch pad 23, a display 24, a speaker 25, a control unit 26, and a data recording unit 27. Note that the host-side device 20 can be configured by, for example, a known mobile terminal or the like owned by the second doctor belonging to a second medical facility 4, so that its detailed description will be omitted.

### (Configuration - host-side device - current location acquisition unit, touch pad, display, speaker)

The current location acquisition unit 21 is current location acquisition unit for detecting a current location of the second doctor on the basis of a GPS signal received from a satellite. The host-side communication unit 22 is host-side communication unit for communicating with the client-side device 10 or the information providing device 30. The touch pad 23 is operation unit for receiving various operation inputs from the second doctor by, for example, being pressed by a finger of the second doctor. The display 24 is display unit for displaying various images on the basis of control by the control unit 26. The speaker 25 is output unit for outputting various voices on the basis of control by the control unit 26. Note that the "display 24" and the "speaker 25" correspond to "host-side output unit" in CLAIMS.

### (Configuration - host-side device - control unit)

The control unit 26 is control unit for controlling the host-side device 20, and specifically, each part of the control unit 26 is substantively configured by installing a host-side program according to the embodiment in the host-side device 20 via any recording medium or the network 2.

Furthermore, the control unit 26 comprises a reply information generation unit 26a in terms of a functional concept as illustrated in Fig. 3. The reply information generation unit 26a is reply information generation unit for generating reply information described below. Note that the detail of the processing executed by the control unit 26 will be described below.

### (Configuration - host-side device - data recording unit)

The data recording unit 27 is recording unit for recording a program necessary to operate the host-side device 20 and various pieces of data (e.g., host information described below of itself).

### (Configuration - information providing device)

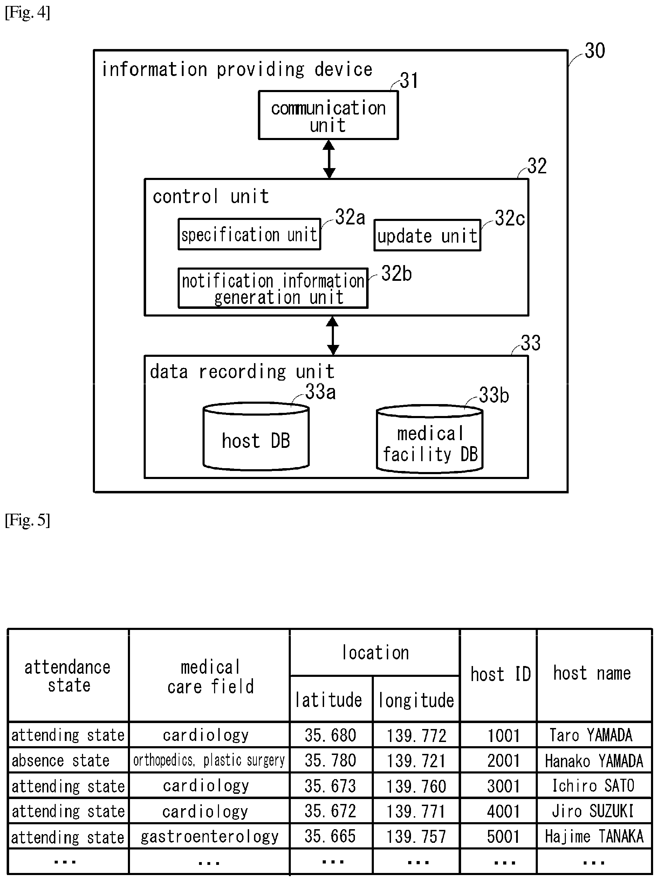

Next, a configuration of the information providing device 30 will be described. Fig. 4 is a block diagram illustrating an electrical configuration of the information providing device 30. The information providing device 30 is an information providing system for transmitting notification information described below, and as illustrated in Fig. 4, is configured to comprise a communication unit 31, a control unit 32, and a data recording unit 33. Note that the information providing device 30 can be configured by, for example, a known server provided in a base station or the like, so that the detailed description thereof will be omitted.

### (Configuration - information providing device - communication unit)

The communication unit 31 is communication unit for communicating with the client-side device 10 or the plurality of host-side devices 20.

### (Configuration - information providing device - control unit)

The control unit 32 is control unit for controlling the information providing device 30, and specifically, each part of the control unit 32 is substantively configured by installing an information providing program according to the embodiment in the information providing device 30 via any recording medium or the network 2.

Furthermore, the control unit 32 comprises a specification unit 32a, a notification information generation unit 32b, and an update unit 32c in terms of a functional concept as illustrated in Fig. 4. The specification unit 32a is specification unit for specifying host information corresponding to retrieval condition information described below from among host information described below stored in a host DB 33a described below. The notification information generation unit 32b is notification information generation unit for generating notification information described below. The update unit 32c is update unit for updating, when doctor current location information described below and medical facility location information described below are acquired by a predetermined method, attendance state information described below stored in the host DB 33a described below on the basis of the doctor current location information and the medical facility location information. Note that the detail of the processing to be executed by the control unit 32 will be described below.

### (Configuration - information providing device - data recording unit)

Data recording unit 33 is recording unit for recording a program necessary to operate the information providing device 30 and various pieces of data, and as illustrated in Fig. 4, comprises the host database 33a (hereinafter, database is referred to as DB), and a medical facility DB 33b.

The host DB 33a is host information storage unit for storing the retrieval condition information and the host information in association with each other. Herein, the "retrieval condition information" is information indicating a condition for retrieving a second doctor (host), and in the embodiment, is a concept including attendance state information described below, medical care field information described below, location information described below, and the like. Furthermore, the "host information" is information for specifying a second doctor (host), and in the embodiment, is a concept including host identification information described below, host name information described below, and the like.

Fig. 5 is a diagram exemplifying the information stored in the host DB 33a. As illustrated in Fig. 5, the host DB 33a stores an item "attendance state", an item "medical care field", an item "location", an item "host ID", and an item "host name", as well as information corresponding to each of the items in association with each other. Among the information, the information corresponding to the item "attendance state" is attendance state information indicating an attendance state of a doctor. Herein, the "attendance state" denotes a state indicating attendance or absence of a doctor, and in the embodiment, description will be made on the assumption that "attending state" is a concept including a state in which a doctor is at work (hereinafter, referred to as an "attendance state"), a state in which a doctor is absent (hereinafter, referred to as an "absence state"), a state in which a doctor is at rest (hereinafter referred to as a "rest state"), and the like. The information corresponding to the item "medical care field" is medical care field information indicating a medical care field of a doctor related to a symptom of a patient. The information corresponding to the item "location" is location information indicating a location of a doctor related to a location of a patient, and in the embodiment, is configured to include latitude of the doctor that is information stored to correspond to an item "latitude", and longitude of the doctor that is information stored to correspond to an item "longitude". The information corresponding to the item "host ID" is host identification information for uniquely identifying a second doctor (host). The information corresponding to the item "host name" is host name information for identifying the name of second doctor (host).

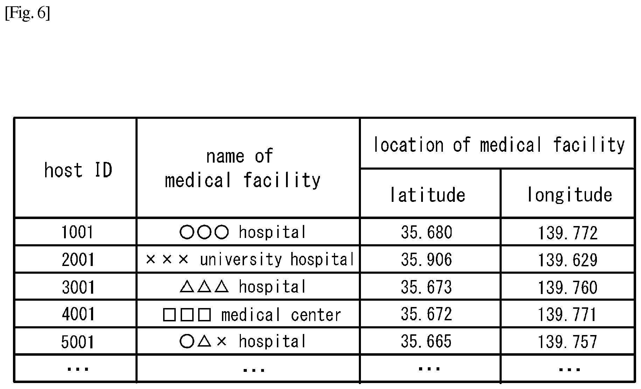

The medical facility DB 33b is medical facility information storage unit for storing information related to a medical facility to which a doctor belongs. Fig. 6 is a diagram exemplifying the information stored in the medical facility DB 33b. As illustrated in Fig. 6, the medical facility dB 33b stores an item "host ID", an item "medical facility name", and an item "medical facility location", as well as information corresponding to each of the items in association with each other. Among the information, the information related to the item "host ID" is host identification information. The information corresponding to the item "medical facility name" is medical facility name information for specifying the name of a medical facility. The information corresponding to the item "medical facility location" is medical facility location information indicating the location of the medical facility, and in the embodiment, is configured to include latitude of the medical facility that is information stored to correspond to an item "latitude", and longitude of the medical facility that is information stored to correspond to an item "longitude".

### (Processing)

Next, the processing to be executed by the retrieval system 1 configured as above will be described.

This processing is roughly divided into retrieval processing and update processing. Hereinafter, each of the retrieval processing and the update processing will be described in detail.

### (Processing - retrieval processing)

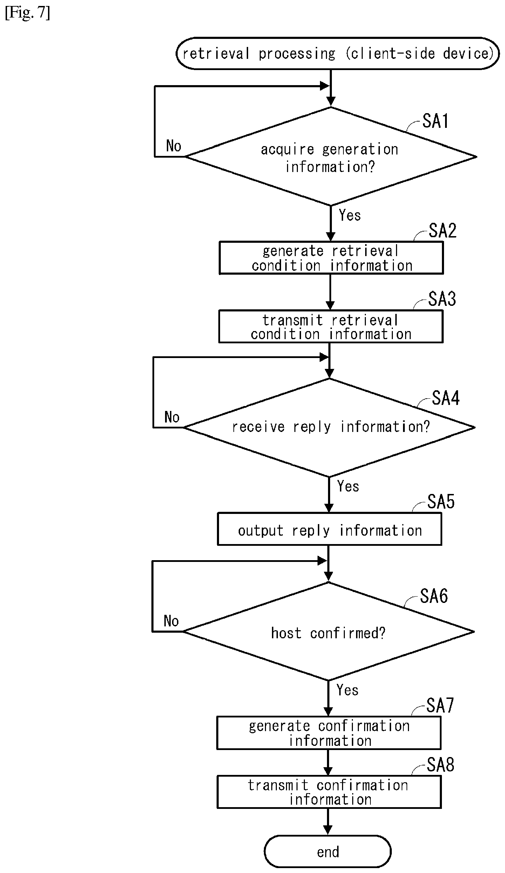

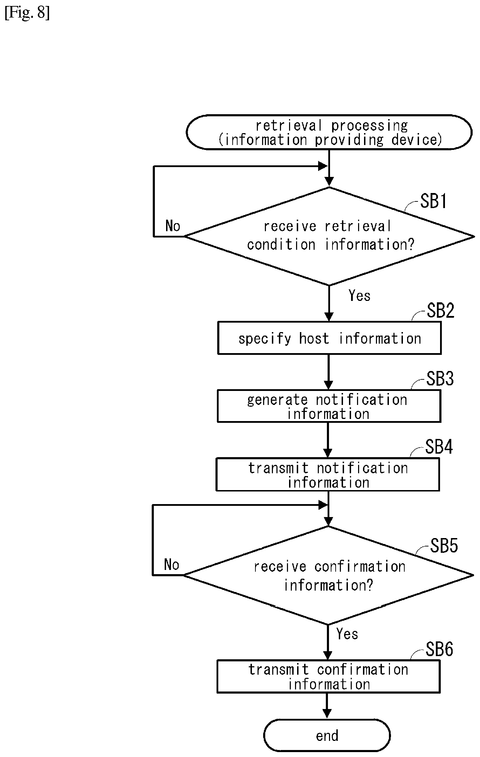

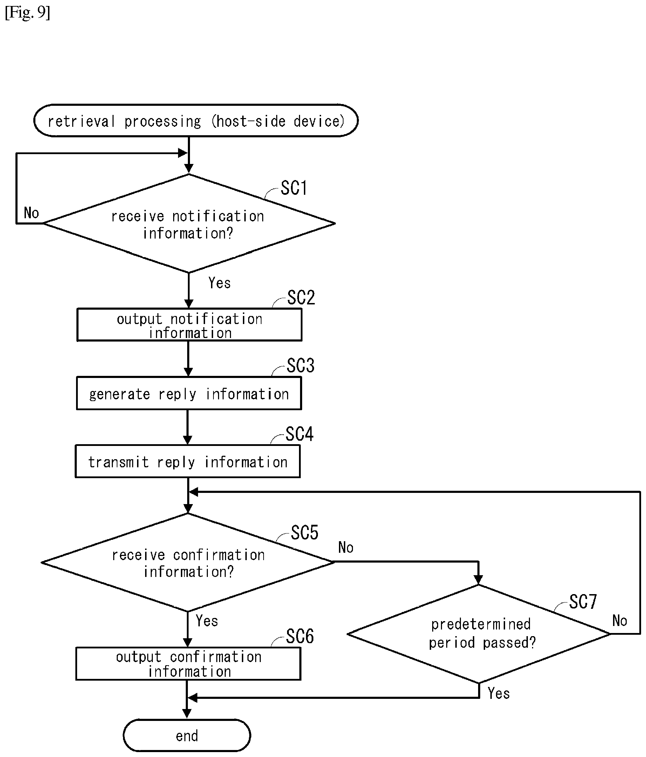

First, the retrieval processing will be described. Fig. 7 is a flowchart of the retrieval processing related to the client-side device 10 according to the embodiment (in the following description of each processing, "step" is abbreviated as "S"). Fig. 8 is a flowchart of the retrieval processing related to the information providing device 30 according to the embodiment. Fig. 9 is a flowchart of the retrieval processing related to the host-side device 20 according to the embodiment. The retrieval processing schematically is processing of retrieving a second doctor (host) who provides a service to a patient (client). Furthermore, although the timing of executing the retrieval processing is optional, in the embodiment, the description will be made on the assumption that the retrieval processing is activated after turning on the power source of the client-side device 10, the plurality of host-side devices 20, and the information providing device 30.

Upon activation of the retrieval processing, as illustrated in Figs. 7 to 9, the control unit 16 of the client-side device 10 performs the processing from SA1 to SA3, the control unit 32 of the information providing device 30 performs the processing from SB 1 to SB4, and the control unit 26 of the host-side device 20 performs the processing from SC1 to SC2 so that notification information described below can be offered to a second doctor.

First, as illustrated in Fig. 7, in SA1, the acquisition unit 16a of the client-side device 10 determines whether the generation information is acquired. Herein, the "generation information" is information for generating the retrieval condition information, and in the embodiment, is a concept including attendance state generation information, medical care field generation information, location generation information and the like. Among the generation information, "attendance state generation information" is information for generating attendance state information among retrieval condition information, and in the embodiment, it will be described as information indicating an attendance state of a second doctor. Furthermore, the "medical care field generation information" is information for generating medical care field information among retrieval condition information, and in the embodiment, it will be described as information indicating a symptom of a patient estimated by a first doctor. Furthermore, the "location generation information" is information for generating the location information among the retrieval condition information, and in the embodiment, it will be described as information indicating the current location of the patient or the first doctor.

Furthermore, although the acquisition method of the generation information is optional, for example, the generation information may be acquired by the method described below. That is, the attendance state generation information among the generation information is acquired by generating a display area (hereinafter, referred to as a "retrieval display area") on a screen of the display 14, displaying the attendance state generation information in the generated retrieval display area as a list, and selecting any one piece of information from among the attendance state generation information via the touch pad 13 by a user when a predetermined timing comes (as an example, when a timing comes that a predetermined operation is received via the touch pad 13 by the first doctor of the client-side device 10). This is not limited thereto, and alternatively, the information input via the touch pad 13 by the first doctor is acquired as the attendance state generation information, or the attendance state generation information is acquired from the data recording unit 17 or an external device (note that, the same applies to the acquisition method of the medical care field generation information). Furthermore, the location generation information among the generation information is acquired by specifying an area of a preset distance range from the current location of the patient or the first doctor (or, a distance range input by the first doctor via the touch pad 13) acquired by the current location acquisition unit 11 from among the area of the map information preliminarily stored in the data recording unit 17, and regarding the information indicating the specified area (for example, information indicating the area within 5 km from the current location of the patient and the like) as the location generation information when the predetermined timing comes.

Then, the acquisition unit 16a of the client-side device 10 waits until the generation information is acquired (SA1, No), and the routine proceeds to SA2 when the generation information is acquired (SA1, Yes).

In SA2, the retrieval condition information generation unit 16b of the client-side device 10 generates the retrieval condition information on the basis of the generation information acquired in SA1. Although the generation method of the retrieval condition information is optional, for example, the retrieval condition information may be generated by the method described below. That is, for the attendance state information among the retrieval condition information, the acquired attendance state generation information is generated as the attendance state information. Furthermore, for the medical care field information among the retrieval condition information, the information indicating the medical care field corresponding to the acquired medical care field generation information (information indicating the symptom of the patient) is specified from a not-shown medical care field table stored in the data recording unit 17, the medical care field table storing symptom information and information indicating a medical care field in association with each other, and the specified information is generated as the medical care field information (as an example, given that the medical care field generation information is "cardiac infarct", the medical care field information is generated as "cardiovascular internal medicine"). This is not limited thereto, and alternatively, the information indicating the medical care field corresponding to the acquired medical care field generation information is acquired from an external device, and the acquired information is generated as the medical care field information. Furthermore, for the location information among the retrieval condition information, the location information corresponding to coordinates in the area indicated by the acquired location generation information (that is, location information indicating the location related to a location of a patient) is extracted from among the location information stored in the host DB 33a of the information providing device 30 (or, location information acquired at a predetermined timing from the host-side device 20 linked to the client-side device 10), and the extracted information is generated as the location information.

In SA3, the control unit 16 of the client-side device 10 allows the client-side communication unit 12 to transmit the retrieval condition information generated in SA2 to the information providing device 30. The transmission method of the retrieval condition information is optional, but in the embodiment, patient-related information is also transmitted with the retrieval condition information. However, this is not limited thereto, and for example, only the retrieval condition information may be transmitted. Herein, "patient-related information" denotes information related to a patient, and is a concept including, for example, information indicating age of the patient, information indicating sex of the patient, information indicating medical history of the patient, information indicating diagnosis of the patient executed in the first medical facility 3 (as an example, image information indicating electrocardiogram), and the like.

Furthermore, as illustrated in Fig. 8, in SB1, the control unit 32 of the information providing device 30 determines whether the retrieval condition information (in the embodiment, the retrieval condition information and the patient-related information) transmitted in SA3 is received by the communication unit 31. Then, the control unit 32 of the information providing device 30 waits until the retrieval condition information is received (SB1, No), and when the retrieval condition information is received, (SB1, Yes), the routine proceeds to SB2.

In SB2, the specification unit 32a of the information providing device 30 specifies the host information corresponding to the retrieval condition information received in SB1 from among the host information (the host identification information, the host name information) stored in the host DB 33a. Herein, the specification method of the host information is optional, but in the embodiment, the host information that matches all the attendance state information, medical care field information, and location information included in the received retrieval condition information is specified from among the host information stored in the host DB 33a. As an example, when the attendance state information included in the received retrieval condition information = "attending state", the medical care field information = "cardiovascular internal medicine", and the location information = "35.680 (latitude), 139.772 (longitude)", "35.673 (latitude), 139.760 (longitude)", "35.672 (latitude), 139.771 (longitude)", "35.665 (latitude), 139.757 (longitude)", host information = "1001 (host identification information), Taro Yamada (host name information)", "3001 (host identification information), Ichiro Sato (host name information)", and "4001 (host identification information), Jiro Suzuki (host name information)" is specified from among the host information in the DB 33a illustrated in Fig. 5. However, this is not limited thereto, and for example, the host information that matches any one piece (or any two pieces) of the attendance state information, the medical care field information, and the location information included in the received retrieval condition information may be specified from among the host information stored in the host DB 33a.

Returning to Fig. 8, in SB3, the notification information generation unit 32b of the information providing device 30 generates the notification information. Herein, the "notification information" denotes information for giving a notice of being the second doctor (host) corresponding to the host information specified in SB2. Furthermore, the generation method of the notification information is optional, and the notification information may be generated by, for example, combining the host information (host identification information, host name information) specified in SB2 and fixed-form message information (text information or voice information) preliminarily stored in the data recording unit 33. As an example, text information or voice information such as "Mr./Ms. ××× is selected as a candidate of host providing medical service" (note that in "×××", the host information (specifically, at least any one of the host identification information and the host name information) is incorporated) is generated.

In SB4, the control unit 32 of the information providing device 30 allows the communication unit 31 to transmit the notification information generated in SB3 to the host-side devices 20 corresponding to the host information specified in SB2. The transmission method of the notification method is optional, but in the embodiment, the patient-related information received in SB 1 is also transmitted with the notification information. However, this is not limited thereto, and for example, only the notification information may be transmitted.

Furthermore, as illustrated in Fig. 9, the control unit 26 of the host-side device 20 determines whether the notification information (in the embodiment, the notification information and the patient-related information) transmitted in SB4 is received by the host-side communication unit 22. Then, the control unit 26 of the host-side device 20 waits until the notification information is received (SC1, No), and when the notification information is received, (SC1, Yes), the routine proceeds to SC2.

In SC2, the control unit 26 of the host-side device 20 allows the display 24 or the speaker 25 output the notification information received in SC1. The output method of the notification information is optional, but for example, a display area (hereinafter, referred to as a "notification display area") may be generated on a screen of the display 24 to continuously or intermittently display the notification information on the generated notification display area, or the notification information may be continuously or intermittently output as a voice by the speaker 25 during a predetermined period from when the notification information is received. Note that in the embodiment, the patient-related information is received with the notification information, so that, for example, the patient-related information and the notification information may be displayed in association with each other in the notification display area.

The above-described processing makes it possible to concurrently transmits the notification information to all the host-side devices 20 that match the retrieval condition information, so that the notification information can be quickly and efficiently offered to the second doctors owning the host-side devices 20.

Next, as illustrated in Fig. 7 and Fig. 9, the control unit 16 of the client-side device 10 executes the processing from SA4 to SA5 and the control unit 26 of the host-side device 20 executes the processing from SC3 to SC 4 so that reply information described below can be offered to the first doctor.

First, as illustrated in Fig. 9, in SC3, the reply information generation unit 26a of the host-side device 20 generates the reply information on the notification information output in SC2. Herein, the "reply information" denotes information indicating the reply of whether medical service (service) can be provided. Furthermore, the generation method of the reply information is optional, but for example, the reply information may be generated by generating a display area (hereinafter, referred to as a "reply generation display area") on the screen of the display 24, displaying, on the generated reply generation display area, the information indicating the reply stating that medical service can be provided and information indicating the reply stating that medical service cannot be provided, selecting (specifying) one piece of information from among the displayed information via the touch pad 23 by the second doctor, and generating the reply information on the basis of the specified information and the host information included in the notification information received in the SC1. As an example, text information or voice information such as "Mr./Ms. ××× can provide medical service" is generated. This is not limited thereto, and alternatively the reply information may be generated on the basis of the information indicating the reply preliminarily stored in the data recording unit 27 (any one of the information indicating the reply stating that medical service can be provided and the information indicating the reply stating that medical service cannot be provided), and some of the information corresponding to the host information included in the notification information.

In SC4, the control unit 26 of the host-side device 20 allows the host-side communication unit 22 to transmit the reply information generated in SC3 to the client-side device 10.

Returning to Fig. 7, in SA4, the control unit 16 of the client-side device 10 determines whether the reply information transmitted in SC4 is received by the client-side communication unit 12. Then, the control unit 16 of the client-side device 10 waits until the reply information is received (SA4, No), and when the reply information is received (SA4, Yes), the routine proceeds to SA5.

In SA5, the control unit 16 of the client-side device 10 allows the display 14 or the speaker 15 to output the reply information received in SA4. The output method of the reply information is optional, but for example, a display area (hereinafter, referred to as a "reply display area") may be generated on the screen of the display 14 to continuously or intermittently display the reply information on the generated reply display area, or the reply information may be continuously or intermittently output as a voice by the speaker 15 during a predetermined period from when the reply information is received.

The above-described processing makes it possible to offer at least one piece of the reply information which the client-side device 10 has received from the host-side devices 20 that match the retrieval condition information to the first doctor (or the patient), so that the second doctor who provides medical service can be determined from among the second doctors corresponding to the offered reply information.

Subsequently, as illustrated in Fig. 7 to Fig. 9, the control unit 16 of the client-side device 10 executes the processing from SA6 to SA 8, the control unit 32 of the information providing device 30 executes the processing from SB5 to SB6, and the control unit 26 of the host-side device 20 executes the processing from SC5 to SC7 so that confirmation information described below can be offered to the second doctor.

First, as illustrated in Fig. 7, the control unit 16 of the client-side device 10 determines whether the second doctor (host) who provides medical service is confirmed in SA6. The confirmation method of the second doctor is optional, but for example, by the first doctor arbitrarily selecting any one piece of information from among the reply information displayed in the reply display area via the touch pad 13 in SA5 (or by the first doctor selecting, via the touch pad 13, the reply information corresponding to the second doctor who has agreed to provide medical service by making contact to, by telephone or the like by the first doctor, at least one of the second doctors corresponding to the reply information from among the displayed reply information), the second doctor corresponding to the selected reply information may be confirmed as the second doctor who should be a confirmation target. Then, the control unit 16 of the client-side device 10 waits until the second doctor is confirmed (SA6, No), and when the second doctor is confirmed (SA6, Yes), the routine proceeds to SA7.

In SA7, the control unit 16 of the client-side device 10 generates the confirmation information. Herein, the "confirmation information" denotes information indicating that the second doctor (host) who provides medical service (service) is confirmed. Furthermore, the generation method of the confirmation information is optional, but for example, the confirmation information may be generated by combining the host information included in the reply information corresponding to the second doctor confirmed in SA6 and the fixed-form message information (text information or voice information) preliminarily stored in the data recording unit 17. As an example, text information or voice information such as "host who provides medical service is confirmed to Mr./Ms. ×××" is generated.

The control unit 16 of the client-side device 10 allows the client-side communication unit 12 to transmit the confirmation information generated in SA7 to the information providing device 30. Upon the transmission, the control unit 16 of the client-side device 10 terminates the retrieval processing.

Furthermore, as illustrated in Fig. 8, in SB5, the control unit 32 of the information providing device 30 determines whether the confirmation information transmitted in SA8 is received by the communication unit 31. Then, the control unit 32 of the information providing device 30 waits until the confirmation information is received (SB5, No), and when the confirmation information is received (SB5, Yes), the routine proceeds to SB6.

In SB6, the control unit 32 of the information providing device 30 allows the communication unit 31 to transmit the confirmation information to at least the other host-side devices 20 other than the host-side device 20 corresponding to the confirmation information received in SB5 from among the host-side devices 20 corresponding to the host information specified in SB2. The transmission method of the confirmation information is optional, but in the embodiment, the confirmation information is transmitted to the host-side devices 20 corresponding to the host information specified in SB2 (that is, the confirmation information is transmitted to the host-side device 20 corresponding to the host information included in the confirmation information and the other host-side devices 20, from among the host-side devices 20 corresponding to the host information specified in SB2). However, this is not limited, and for example, as described above, when the confirmation information includes the information indicating that agreement about providing medical service has obtained with the second doctor who should be a confirmation target by a telephone or the like in SA 6, another piece of the host information other than the piece of the host information included in the confirmation information received in SB5 may be specified from among the host information specified in SB 2 to transmit the confirmation information only to the host-side device 20 corresponding to the specified host information. Upon the transmission, the control unit 32 of the information providing device 30 terminates the retrieval processing.

Furthermore, as illustrated in Fig. 9, in SC5, the control unit 26 of the host-side device 20 determines whether the confirmation information transmitted in SB6 is received by the host-side communication unit 22. Then, the control unit 26 of the host-side device 20 allows the routine to proceed to SC6 when it is determined that the confirmation information is received (SC5, Yes), or to SC7 when it is determined that the confirmation information is not received (SC5, No).

In SC6, the control unit 26 of the host-side device 20 allows the display 24 or the speaker 25 to output the confirmation information received in SC5. The output method of the confirmation information is optional, but for example, a display area (hereinafter, referred to as "confirmation display area") may be generated on the screen of the display 24 to continuously or intermittently display the confirmation information in the generated confirmation display area, or the confirmation information may be continuously or intermittently output as a voice by the speaker 25 during a predetermined period from when the confirmation information is received. Then, the control unit 26 of the host-side device 20 terminates the retrieval processing.

In SC7, the control unit 26 of the host-side device 20 determines whether a predetermined period has passed from when the processing of SC4 is performed. Then, the control unit 26 of the host-side device 20 allows the routine to proceed to SC5 when it is not determined that the predetermined time has passed (SC7, No), or terminates the retrieval processing when it is determined that the predetermined time has passed (SC7, Yes).

The above-described processing makes it possible to offer the confirmation information to the other second doctors who do not provide medical service to the patient (client) among the second doctors (hosts) of the respective host side devices 20 specified in SB2, so that the other second doctors can afterward confirm that medical service has provided to the patient by a second doctor other than he/she.

Furthermore, such retrieval processing makes it possible to quickly and efficiently retrieve the second doctor (host) who matches the needs of the patient (client) as compared with a conventional technique, so that retrieval performance of the second doctor can be improved.

### (Processing - update processing)

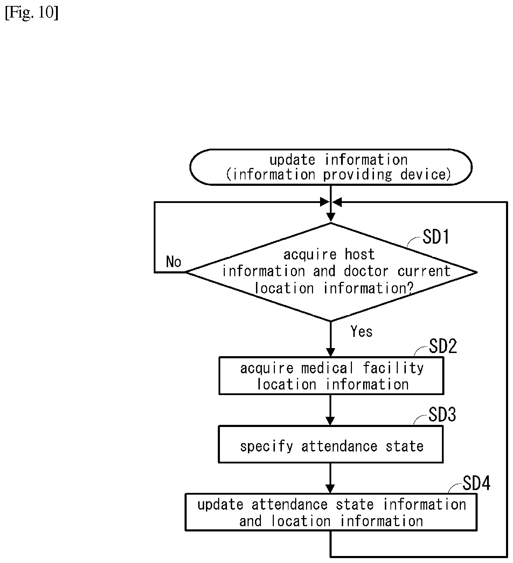

First, the update processing will be described. Fig. 10 is a flowchart of the update processing related to the information providing device 30 according to the embodiment. The update processing is processing to update the retrieval condition information (particularly, the attendance state information and the location information) stored in the host DB 33a of the information providing device 30. Furthermore, the timing of executing the update processing is optional, but in the embodiment, the description will be made on the assumption that the update processing is activated after turning on the power source of the information providing device 30 and at least one of the plurality of host-side devices 20 and is executed along with the retrieval processing.

Upon activation of the update processing, as illustrated in Fig. 10, in SD1, the control unit 32 of the information providing device 30 determines whether the host information and the doctor current location information related to the host-side device 20 transmitted from the host-side device 20 at a predetermined timing are acquired (received). Herein, the "doctor current location information" denotes information indicating the current location of the second doctor acquired by the current location acquisition unit 21 of the host-side device 20. Then, the control unit 32 of the information providing device 30 waits until the host information and the doctor current location information are acquired (SD1, No), and when the host information and the doctor current location information are acquired (SD1, Yes), the routine proceeds to SD2.

The control unit 32 of the information providing device 30 acquires the medical facility location information corresponding to the host information acquired in SD1 from among the medical facility location information stored in the medical facility DB 33b.

In SD3, the control unit 32 of the information providing device 30 specifies the attendance state of the second doctor corresponding to the host information acquired in SD1. The specification method of the attendance state is optional, but in the embodiment, the attendance state is specified on the basis of the doctor current location information acquired in SD1 and the medical facility location information acquired in SD 2. Specifically, it is determined whether the location of the second doctor of the doctor current location information is located within a predetermined distance (e.g., within 500 m) from the location of the medical facility of the medical facility location information, and it is specified that the second doctor is in the attending state when it is determined that the second doctor is located within the predetermined distance, and it is specified that the second doctor is in the absence state when it is determined that the second doctor is located out of the predetermined distance.

In SD4, the update unit 32c of the information providing device 30 updates the attendance state information and the location information stored in the host DB 33a of the information providing device 30 on the basis of the doctor current location information acquired in SD1 and the medical facility location information acquired in SD2. Herein, for the update method of the attendance state information, in the embodiment, the information indicating the attendance state of the second doctor specified in SD3 is updated as the attendance state information corresponding to the host information acquired in SD1 among the attendance state information stored in the host DB 33a. Furthermore, for the update method of the location information, the doctor current location information acquired in SD1 is updated as the location information corresponding to the host information acquired in SD 1 among the location information stored in the host DB 33a. Then, the control unit 32 of the information providing device 30 allows the routine to proceed to SD1.

Such update processing makes it possible to automatically update the attendance state information and the location information stored in the host DB 33a. Particularly, the attendance state information can be updated without receiving a notification indicating an attendance state from a second doctor (host), so that a trouble of an operation related to the update by the second doctor can be eliminated.

### (Effects of embodiment)

As described above, according to the embodiment, the client-side communication unit 12 of the client-side device 10 transmits the retrieval condition information generated by the retrieval condition information generation unit 16b to the information providing device 30, the specification unit 32a of the information providing device 30 specifies the host information corresponding to the retrieval condition information from among the host information stored in the host dB 33a when the retrieval condition information is received by the communication unit 31, and the communication unit 31 of the information providing device 30 transmits the notification information generated by the notification information generation unit 32b to the host-side devices 20 corresponding to the host information specified by the specification unit 32a, so that the notification information can be transmitted to all the host-side devices 20 that match the retrieval condition information, making it possible to quickly and efficiently offer the notification information to the second doctors (hosts) of the host-side devices 20. Furthermore, the host-side communication unit 22 of each of the plurality of host-side devices 20 transmits the reply information generated by the reply information generation unit 26a to the client-side device 10, and the display 14 or the speaker 15 of the client-side device 10 outputs the reply information when the client-side communication unit 12 receives the reply information, so that the client-side device 10 can offer at least one piece of the reply information received from the host-side devices 20 that match the retrieval condition information to the patient (client) or the first doctor, making it possible to determine the second doctor who provides medical service from among the second doctors corresponding to the reply information. This makes it possible to quickly and effectively retrieve the second doctor who matches the needs of the patient as compared with a conventional technique, so that the retrieval performance can be improved.

Furthermore, the service is medical service, and the retrieval condition information includes the attendance state information indicating an attendance state of a doctor, the medical care field information indicating a medical care field of a doctor related to a symptom of a patient, or the location information indicating a location of a doctor related to a location of a patient, so that the host that matches the needs of the patient (client) who receives medical service can be retrieved even more efficiently, making it possible to further improve the retrieval performance of the second doctor (host).

Furthermore, the information providing device 30 is equipped with the update unit 32c that updates, when the doctor current location information and the medical facility location information are acquired by a predetermined method, the attendance state information among the retrieval condition information stored in the host DB 33a on the basis of the doctor current location information and the medical facility location information, so that the attendance state information stored in the host DB 33a can be updated without receiving a notification indicating attendance state from the second doctor (host), making it possible to eliminate a trouble of an operation related to the update by the second doctor.

Furthermore, the communication unit 31 of the information providing device 30 transmits, when the confirmation information is received by the communication unit 31 from the client-side device 10, the confirmation information to the host-side devices 20 other than the host-side device 20 corresponding to the confirmation information among the host-side devices 20 corresponding to the host information specified by the specification unit 32a, so that the confirmation information can be offered to the other second doctors who do not provide service to the patient (client) among the second doctors (hosts) of the host-side devices 20 specified by the specification unit 32a, enabling the other second doctors to afterward confirm that service has provided to the patient by a second doctor other than he/she.

### (Variation of embodiment)

While the embodiment according to the invention is described above, the specific configuration and unit of the invention can be altered and modified arbitrarily within the scope of the technical idea of each invention described in CLAIMS. Hereinafter, such variations will be described.

### (Problems to be solved and advantageous effects of invention)

First, problems to be solved and advantageous effects of invention are not limited to the above-described content, and may be differ according to the execution environment or the detailed configuration of the invention, and only some of the above problems may be solved or only some of the above-described effects may be provided.

### (Distribution and integration)

Each of the electrical constituent elements described above is of a functional concept, and is not necessarily needed to be physically configured as illustrated in the drawings. That is, the specific form of disintegration and integration of each part is not limited to the one illustrated in the drawings, and some or all thereof can be configured by functional and physical disintegration and integration by any unit depending on various loads, using state, or the like. "System" in the present application is not limited to the one configured by a plurality of devices, and includes the one configured by a single device. Furthermore, "device" in the present application is not limited to the one configured by a single device, and includes the one configured by a plurality of devices. For example, the information providing device 30 may be configured by distributing it into a plurality of devices configured to be communicable with each other and providing the control unit 32 in some of the plurality of devices and the data recording unit 33 in others of the plurality of devices.

### (Shape, numeral value, structure, and time series)

Regarding the constituent elements exemplified in the embodiment or in the figures, the shape, numeral values or structures of a plurality of the constituent elements or a mutual relationship in a time series may be arbitrarily modified and improved within a range of the technical idea of the present invention.

### (Application target)

In the above embodiment, it was described that the retrieval system 1 is applied to a case of retrieving a second doctor capable of providing medical service to a patient urgently transported to the first medical facility 3, but this is not limited thereto. For example, the retrieval system 1 may be applied to a case where a symptom is found that cannot be treated in the first medical facility 3 as a result of diagnosing a patient hospitalized in the first medical facility 3 and a second doctor capable of providing medical service to the patient is retrieved. Alternatively, the retrieval system 1 may be applied to a case where a patient hospitalized in the first medical facility 3 check outs the first medical facility 3, and the patient retrieves a second doctor belonging to a second facility 4 (e.g., medical clinic, etc.) to which the patient can go.

### (Second Doctor)

In the above embodiment, it was described that the second doctor is a doctor belonging to a medical facility different from the medical facility to which the first doctor belongs, but this is not limited thereto, and for example, the second doctor may be a doctor belonging to the same medical facility to which the first doctor belongs.

### (Retrieval condition information)

In the above embodiment, it was described that the retrieval condition information includes the attendance state information, the medical care field information, and the location information, but this is not limited thereto. For example, including information indicating evaluation of a doctor, information indicating the scale of the medical facility to which a doctor belongs, and information indicating the number of times of medical care undergone by a patient from a doctor in addition to the attendance state information, the medical care field information, and the location information makes it possible to even more efficiently retrieve the second doctor who matches the needs of the patient (client).

### (Client-side device)

In the above embodiment, it was described that the client-side device 10 is owned by the first doctor, but this is not limited thereto, and for example, the client-side device 10 may be owned by a patient, or may be owned by a staff (client-side assistant) of an ambulance that transports a patient.

### (Host-side device)

In the above embodiment, it was described that the host-side device 20 is owned by the second doctor, but this is not limited thereto, and for example, the host-side device 20 may be owned by a nurse (host-side assistant) or a clerk (host-side assistant) of the medical facility to which the second doctor belongs.

### (Retrieval processing)

In the above embodiment, it was described that the processing from SA6 to SA8, from SB5 to SB6, and from SC5 to SC7 is performed, but the processing from SA6 to SA8, from SB5 to SB6, and from SC5 to SC7 may be omitted to reduce the processing load of the retrieval system 1. In this case, for example, the second doctor who provides medical service may be confirmed by the second doctor of the host-side device 20 negotiating with the first doctor of the client-side device using a telephone or the like after the processing of SC4.

Furthermore, in the above embodiment, it was described that the notification information generated in SB 3 is transmitted to all the host-side devices 20 corresponding to the host information specified in SB 2 in SB4, but this is not limited thereto, and the notification information may be transmitted to only some of the host-side devices 20 corresponding to the host information specified in SB2. Specifically, first, the control unit 32 of the information providing device 30 allows the communication unit 31 to transmit, after the host information corresponding to the retrieval condition information received in SB1 is specified, the specified host information and the retrieval condition information corresponding to the specified host information to the client-side device 10 in SB2. Then, the control unit 16 of the client-side device 10 allows the display 14 to output the host information and the retrieval condition information received from the information providing device 30 (for example, to display the host information and the retrieval condition information in association with each other as a list, or to display the host information (e.g., an icon image) at a position of the location information corresponding to the host information on a map). Next, the control unit 16 of the client-side device 10 specifies, from among the displayed host information, the host information selected via the touch pad 13 as host information corresponding to a host to which the notification information should be transmitted, and allows the client-side communication unit 12 to transmit the specified host information to the client-side device 10. Then, the control unit 32 of the information providing device 30 allows the communication unit 31 to transmit the notification information generated in SB3 to the host-side device 20 corresponding to the host information specified by the client-side device 10. This makes it possible to even more efficiently retrieve the second doctor who matches the needs of the patient (client).

Furthermore, in the above embodiment, it was described that the reply information generated in SC3 is directly transmitted to the client-side device 10 in SC4, but this is not limited thereto, and for example, the reply information generated in SC 3 may be transmitted to the client-side device 10 via the information providing device 30.

### (Update processing)

In the above embodiment, it was described that the update processing is executed, but this is not limited thereto, and for example, the update processing may be omitted. In this case, the update unit 32c of the information providing device 30 may be also omitted.

Furthermore, in the above embodiment, it was described that the attendance state information and the location information stored in the host DB 33a of the information providing device 30 are updated, but this is not limited thereto, and for example, only the attendance state information may be updated. Furthermore, in the above embodiment, it was described that the attendance state information is updated on the basis of the doctor current location information acquired in SD1 and the medical facility location information acquired by SD2, but this is not limited thereto, and for example, the information transmitted from the host-side device 20 and indicating the attendance state input via the touch pad 23 by the second doctor may be updated as the attendance state information.

### (Notes)

A retrieval system according to note 1 is a retrieval system for retrieving a host that provides a service to a client, the retrieval system comprising a client-side device, a plurality of host-side devices, and an information providing system communicably connected to the client-side device and each of the plurality of host-side devices, wherein the client-side device comprises: client-side communication unit for communicating with the plurality of host-side devices or the information providing system; acquisition unit for acquiring generation information for generating retrieval condition information indicating a condition for retrieving the host; retrieval condition information generation unit for generating the retrieval condition information; client-side output unit for outputting various pieces of information related to the client-side device; and a medical care field table storing symptom information indicating a symptom of a patient and information indicating a medical care field in association with each other, each of the plurality of host-side devices comprises: host-side communication unit for communicating with the client-side device or the information providing system; reply information generation unit for generating reply information indicating a reply of whether the service can be provided; and host-side output unit for outputting various pieces of information related to the each of the host-side devices, and the information providing system comprises: communication unit for communicating with the client-side device or the plurality of host-side devices; host information storage unit for storing the retrieval condition information and host information for specifying the host in association with each other; specification unit for specifying the host information corresponding to the retrieval condition information from among the host information stored in the host information storage unit when the retrieval condition information is received by the communication unit; and notification information generation unit for generating notification information for giving a notice of being a host corresponding to the host information specified by the specification unit, and wherein the retrieval condition information generation unit of the client-side device generates the retrieval condition information based on the generation information when the generation information is acquired by the acquisition unit, the client-side communication unit of the client-side device transmits the retrieval condition information generated by the retrieval condition information generation unit to the information providing system, the specification unit of the information providing system specifies the host information corresponding to the retrieval condition information from among the host information stored in the host information storage unit when the retrieval condition information is received by the communication unit, the communication unit of the information providing system transmits the notification information generated by the notification information generation unit to one or more of the host-side devices corresponding to the host information specified by the specification unit, the host-side output unit of each of the plurality of host-side devices outputs the notification information when the notification information is received by the host-side communication unit, the reply information generation unit of each of the plurality of host-side devices generates the reply information on the notification information output by the host-side output unit and that indicates the reply specified by a predetermined method, the host-side communication unit of each of the plurality of host-side devices transmits the reply information generated by the reply information generation unit to the client-side device or the information providing system, the client-side output unit of the client-side device outputs the reply information when the reply information is received by the client-side communication unit, the service is a medical service, the retrieval condition information includes medical care field information indicating a medical care field of the doctor related to the symptom of the patient and attendance state information indicating an attendance state of the doctor, or location information indicating a location of the doctor related to location of the patient, the generation information includes medical care field generation information for generating the medical care field information, the medical care field generation information indicating the symptom of the patient, and attendance state generation information for generating the attendance state information, the attendance state generation information indicating the attendance state of the doctor, or location generation information for generating the location information, the location generation information indicating an area in a predetermined distance range from a current location of the patient or a client assistant, and the retrieval condition information generation unit of the client-side device specifies, when the medical care field generation information is acquired by the acquisition unit, information indicating the medical care field corresponding to the medical care field generation information from among information indicating the medical care field stored in the medical care field table, and generates the specified information as the medical care field information, generates, when the attendance state generation information is acquired by the acquisition unit, the attendance state generation information as the attendance state information, and extracts, when the location generation information is acquired by the acquisition unit, the location information corresponding to coordinates in the area indicated by the location generation information from among the location information acquired by the information providing system or the host-side devices, and generates the extracted information as the location information.

A retrieval system according to note 2 is a retrieval system for retrieving a host that provides a service to a client, the retrieval system comprising a client-side device, a plurality of host-side devices, and an information providing system communicably connected to the client-side device and each of the plurality of host-side devices, wherein the client-side device comprises: client-side communication unit for communicating with the plurality of host-side devices or the information providing system; acquisition unit for acquiring generation information for generating retrieval condition information indicating a condition for retrieving the host; retrieval condition information generation unit for generating the retrieval condition information; and client-side output unit for outputting various pieces of information related to the client-side device, each of the plurality of host-side devices comprises: host-side communication unit for communicating with the client-side device or the information providing system; reply information generation unit for generating reply information indicating a reply of whether the service can be provided; and host-side output unit for outputting various pieces of information related to the each of the host-side devices, and the information providing system comprises: communication unit for communicating with the client-side device or the plurality of host-side devices; host information storage unit for storing the retrieval condition information and host information for specifying the host in association with each other; specification unit for specifying host information corresponding to the retrieval condition information from among the host information stored in the host information storage unit when the retrieval condition information is received by the communication unit, and notification information generation unit for generating notification information for giving a notice of being a host corresponding to the host information specified by the specification unit, and wherein the retrieval condition information generation unit of the client-side device generates the retrieval condition information based on the generation information when the generation information is acquired by the acquisition unit, the client-side communication unit of the client-side device transmits the retrieval condition information generated by the retrieval condition information generation unit to the information providing system, the specification unit of the information providing system specifies the host information corresponding to the retrieval condition information from among the host information stored in the host information storage unit when the retrieval condition information is received by the communication unit, the communication unit of the information providing system transmits the notification information generated by the notification information generation unit to one or more of the host-side devices corresponding to the host information specified by the specification unit, the host-side output unit of each of the plurality of host-side devices outputs the notification information when the notification information is received by the host-side communication unit, the reply information generation unit of each of the plurality of host-side devices generates the reply information on the notification information output by the host-side output unit and that indicates the reply specified by a predetermined method, the host-side communication unit of each of the plurality of host-side devices transmits the reply information generated by the reply information generation unit to the client-side device or the information providing system, the client-side output unit of the client-side device outputs the reply information when the reply information is received by the client-side communication unit, the service is a medical service, the retrieval condition information includes attendance state information indicating an attendance state of the doctor, or location information indicating a location of the doctor related to a location of the patient, the generation information includes attendance state generation information for generating the attendance state information, the attendance state generation information indicating the attendance state of the doctor, or location generation information for generating the location information, the location generation information indicating an area in a predetermined distance range from a current location of the patient or a client assistant, and the retrieval condition information generation unit of the client-side device generates, when the attendance state generation information is acquired by the acquisition unit, the attendance state generation information as the attendance state information, and extracts, when the location generation information is acquired by the acquisition unit, the location information corresponding to coordinates in the area indicated by the location generation information from among the location information acquired by the information providing system or the host-side devices, and generates the extracted information as the location information.

An information providing system according to note 3 is an information providing system communicably connected to a client-side device and each of a plurality of host-side devices, the information providing system comprising: communication unit for communicating with the client-side device or the plurality of host-side devices; host information storage unit for storing retrieval condition information indicating a condition for retrieving a host that provides a service to a client, and host information for specifying the host in association with each other; specification unit for specifying host information corresponding to the retrieval condition information from among the host information stored in the host information storage unit when the retrieval condition information is received by the communication unit; and notification information generation unit for generating notification information for giving a notice of being a host corresponding to the host information specified by the specification unit, wherein the specification unit specifies the host information corresponding to the retrieval condition information from among the host information stored in the host information storage unit when by the communication unit receives the retrieval condition information from the client-side device, the communication unit transmits the notification information generated by the notification information generation unit to one or more of the host-side devices corresponding to the host information specified by the specification unit, the service is a medical service, and the retrieval condition information includes location information indicating a location of the doctor related to a location of the patient.

In accordance with the invention according to note 3, the information providing system according to note 4 comprising update unit for updating, when doctor current location information indicating a current location of the doctor and medical facility location information indicating a location of a medical facility to which the doctor belongs are acquired by a predetermined method, the attendance state information among the retrieval condition information stored in the host information storage unit based on the doctor current location information and the medical facility location information.

In accordance with the invention according to note 3 or note 4, the information providing system according to note 5, wherein the communication unit of the information providing system transmits, when the communication unit receives confirmation information indicating that the host that provides the service has confirmed from the client-side device, the confirmation information to one or more of the host-side devices other than one of the host-side devices corresponding to the confirmation information from among some of the host-side devices corresponding to the host information specified by the specification unit.

A client-side device according to note 6 is a client-side device communicably connected to an information providing device and each of a plurality of host-side devices, the client-side device comprising: client-side communication unit for communicating with the plurality of host-side devices or the information providing system; acquisition unit for acquiring generation information for generating retrieval condition information indicating a condition for retrieving a host; retrieval condition information generation unit for generating the retrieval condition information; client-side output unit for outputting various pieces of information related to the client-side device; and a medical care field table storing symptom information indicating a symptom of a patient and information indicating a medical care field in association with each other, wherein the retrieval condition information generation unit generates the retrieval condition information based on the generation information when the generation information is acquired by the acquisition unit, the client-side communication unit transmits the retrieval condition information generated by the retrieval condition information generation unit to the information providing system, the client-side output unit outputs, when the client-side communication unit receives reply information indicating a reply of whether a service can be provided to a client, the reply information, the service is a medical service, the retrieval condition information includes medical care field information indicating a medical care field of the doctor related to the symptom of the patient and attendance state information indicating an attendance state of the doctor, or location information indicating a location of the doctor related to location of the patient, the generation information includes medical care field generation information for generating the medical care field information, the medical care field generation information indicating the symptom of the patient, and attendance state generation information for generating the attendance state information, the attendance state generation information indicating the attendance state of the doctor, or location generation information for generating the location information, the location generation information indicating an area in a predetermined distance range from a current location of the patient or a client assistant, and the retrieval condition information generation unit specifies, when the medical care field generation information is acquired by the acquisition unit, information indicating the medical care field corresponding to the medical care field generation information from among information indicating the medical care field stored in the medical care field table, and generates the specified information as the medical care field information, and extracts, when the location generation information is acquired by the acquisition unit, the location information corresponding to coordinates in the area indicated by the location generation information from among the location information acquired by the information providing system or the host-side devices, and generates the extracted information as the location information.

A client-side device according to note 7 is a client-side device communicably connected to an information providing device and each of a plurality of host-side devices, the client-side device comprising: client-side communication unit for communicating with the plurality of host-side devices or the information providing system; acquisition unit for acquiring generation information for generating retrieval condition information indicating a condition for retrieving a host; retrieval condition information generation unit for generating the retrieval condition information; client-side output unit for outputting various pieces of information related to the client-side device; and a medical care field table storing symptom information indicating symptom of a patient and information indicating a medical care field in association with each other, wherein the retrieval condition information generation unit generates the retrieval condition information based on the generation information when the generation information is acquired by the acquisition unit, the client-side communication unit transmits the retrieval condition information generated by the retrieval condition information generation unit to the information providing system, the client-side output unit outputs, when the client-side communication unit receives reply information indicating a reply of whether a service can be provided to a client, the reply information, the service is a medical service, the retrieval condition information includes attendance state information indicating an attendance state of a doctor, or location information indicating a location of the doctor related to a location of the patient, the generation information includes attendance state generation information for generating the attendance state information, the attendance state generation information indicating the attendance state of the doctor, or location generation information for generating the location information, the location generation information indicating an area in a predetermined distance range from a current location of the patient or a client assistant, and the retrieval condition information generation unit of the client-side device generates, when the attendance state generation information is acquired by the acquisition unit, the attendance state generation information as the attendance state information, and extracts, when the location generation information is acquired by the acquisition unit, the location information corresponding to coordinates in the area indicated by the location generation information from among the location information acquired by the information providing system or the host-side devices, and generates the extracted information as the location information.

An information providing method according to note 8 is an information providing method performed in an information providing system communicably connected to a client-side device and each of a plurality of host-side devices, the information providing method comprising: a communication step for communication unit communicating with the client-side device or the plurality of host-side devices; a specification step for specification unit specifying, when retrieval condition information indicating a condition for retrieving a host that provides a service to a client is received by the communication unit, host information corresponding to the received retrieval condition information from among host information for specifying the host stored in host information storage unit with the retrieval condition information in association to each other; and a notification information generation step for notification information generation unit generating notification information for giving a notice of being a host corresponding to the host information specified in the specification step; wherein in the specification step, the host information corresponding to the retrieval condition information is specified from among the host information stored in the host information storage unit when the retrieval condition information is received from the client-side device in the communication step, in the communication step, the notification information generated in the notification information generation step is transmitted to one or more of the host-side devices corresponding to the host information specified in the specification step, the service is a medical service, and the retrieval condition information includes location information indicating a location of the doctor related to a location of the patient.

An information providing program according to note 9 is an information providing program for being executed by an information providing system communicably connected to a client-side device and each of a plurality of host-side devices, the information providing program causing a computer to function as: communication unit for communicating with the client-side device or the plurality of host-side devices; host information storage unit for storing retrieval condition information indicating a condition for retrieving a host that provides a service to a client and host information for specifying the host in association with each other; specification unit for specifying host information corresponding to the retrieval condition information from among the host information stored in the host information storage unit when the retrieval condition information is received by the communication unit; and notification information generation unit for generating notification information for giving a notice of being a host corresponding to the host information specified by the specification unit, wherein the specification unit specifies the host information corresponding to the retrieval condition information from among the host information stored in the host information storage unit when the communication unit receives the retrieval condition information from the client-side device, the communication unit transmits the notification information generated by the notification information generation unit to one or more of the host-side devices corresponding to the host information specified by the specification unit, the service is a medical service, and the retrieval condition information includes location information indicating a location of the doctor related to a location of the patient.

A client-side program according to note 10 is a client-side program for being executed by a client-side device communicably connected to an information providing system and each of a plurality of host side devices, the client-side program causing a computer to function as: client-side communication unit for communicating with the plurality of host-side devices or the information providing system; acquisition unit for acquiring generation information for generating retrieval condition information indicating a condition for retrieving a host; retrieval condition information generation unit for generating the retrieval condition information; client-side output unit for outputting various pieces of information related to the client-side device; and a medical care field table storing symptom information indicating a symptom of a patient and information indicating a medical care field in association with each other, wherein the retrieval condition information generation unit generates the retrieval condition information based on the generation information when the generation information is acquired by the acquisition unit, the client-side communication unit transmits the retrieval condition information generated by the retrieval condition information generation unit to the information providing system, the client-side output unit outputs, when the client-side communication unit receives reply information indicating a reply of whether a service can be provided to a client, the reply information, the service is a medical service, the retrieval condition information includes medical care field information indicating a medical care field of the doctor related to the symptom of the patient, and attendance state information indicating an attendance state of a doctor, or location information indicating a location of the doctor related to location of the patient, the generation information includes medical care field generation information for generating the medical care field information, the medical care field generation information indicating the symptom of the patient, and attendance state generation information for generating the attendance state information, the attendance state generation information indicating the attendance state of the doctor, or location generation information for generating the location information, the location generation information indicating an area in a predetermined distance range from a current location of the patient or a client assistant, and the retrieval condition information generation unit specifies, when the medical care field generation information is acquired by the acquisition unit, information indicating the medical care field corresponding to the medical care field generation information from among information indicating the medical care field stored in the medical care field table, and generates the specified information as the medical care field information, generates, when the attendance state generation information is acquired by the acquisition unit, the attendance state generation information as the attendance state information, and extracts, when the location generation information is acquired by the acquisition unit, the location information corresponding to coordinates in the area indicated by the location generation information from among the location information acquired by the information providing system or the host-side devices, and generates the extracted information as the location information.

A client-side program according to note 11 is a client-side program for being executed by a client-side device communicably connected to an information providing system and each of a plurality of host side devices, the client-side program causing a computer to function as: client-side communication unit for communicating with the plurality of host-side devices or the information providing system; acquisition unit for acquiring generation information for generating retrieval condition information indicating a condition for retrieving a host; retrieval condition information generation unit for generating the retrieval condition information; client-side output unit for outputting various pieces of information related to the client-side device; and a medical care field table storing symptom information indicating symptom of a patient and information indicating a medical care field in association with each other, wherein the retrieval condition information generation unit generates the retrieval condition information based on the generation information when the generation information is acquired by the acquisition unit, the client-side communication unit transmits the retrieval condition information generated by the retrieval condition information generation unit to the information providing system, the client-side output unit outputs, when the client-side communication unit receives reply information indicating a reply of whether a service can be provided to a client, the reply information, the service is a medical service, the retrieval condition information includes attendance state information indicating an attendance state of a doctor, or location information indicating a location of the doctor related to a location of the patient, the generation information includes attendance state generation information for generating the attendance state information, the attendance state generation information indicating the attendance state of the doctor, or location generation information for generating the location information, the location generation information indicating an area in a predetermined distance range from a current location of the patient or a client assistant, and the retrieval condition information generation unit of the client-side device generates, when the attendance state generation information is acquired by the acquisition unit, the attendance state generation information as the attendance state information, and extracts, when the location generation information is acquired by the acquisition unit, the location information corresponding to coordinates in the area indicated by the location generation information from among the location information acquired by the information providing system or the host-side devices, and generates the extracted information as the location information.

### (Effects of notes)

According to the retrieval system of note 1, the information providing system of note 3, the client-side device of note 6, the information providing method of note 8, the information providing program of note 9, and the client-side program of note 10, the client-side communication unit of the client-side device transmits the retrieval condition information generated by the retrieval condition information generation unit to the information providing device, the specification unit of the information providing device specifies the host information corresponding to the retrieval condition information from among the host information stored in the host information storing unit when the retrieval condition information is received by the communication unit, and the communication unit of the information providing device transmits the notification information generated by the notification information generation unit to the host-side devices corresponding to the host information specified by the specification unit, so that the notification information can be transmitted to all the host-side devices that match the retrieval condition information, making it possible to quickly and efficiently offer the notification information to the hosts of the host-side devices. Furthermore, the host-side communication unit of each of the plurality of host-side devices transmits the reply information generated by the reply information generation unit to the client-side device, and the output device of the client-side device outputs the reply information when the client-side communication unit receives the reply information, so that the client-side device can offer at least one piece of the reply information received from the host-side devices that match the retrieval condition information to the client, making it possible to determine the host who provides medical service from among the hosts corresponding to the reply information. This makes it possible to quickly and effectively retrieve the host who matches the needs of the client as compared with a conventional technique, so that the retrieval performance can be improved.

Furthermore, the service is medical service, and the retrieval condition information includes the attendance state information indicating an attendance state of a doctor, the medical care field information indicating a medical care field of a doctor related to a symptom of a patient, or the location information indicating a location of a doctor related to a location of a patient, so that the host that matches the needs of the client who receives medical service can be retrieved even more efficiently, making it possible to further improve the retrieval performance of the host.

Furthermore, the retrieval condition information includes attendance state information indicating an attendance state of the doctor, or location information indicating a location of the doctor related to a location of the patient, so that the host that matches the needs of the client who receives medical service can be retrieved even more efficiently, making it possible to further improve the retrieval performance of the host.

According to the retrieval system of note 2, the client-side device of note 7, and the client-side program of note 11, the service is medical service, and the retrieval condition information includes the attendance state information indicating an attendance state of a doctor, or the location information indicating a location of a doctor related to a location of a patient, so that the host that matches the needs of the client who receives medical service can be retrieved even more efficiently, making it possible to further improve the retrieval performance of the host.

According to the information providing device of note 4, the information providing device is equipped with the update unit that updates, when the doctor current location information and the medical facility location information are acquired by a predetermined method, the attendance state information among the retrieval condition information stored in the host information storing unit on the basis of the doctor current location information and the medical facility location information, so that the attendance state information stored in the host information storing unit can be updated without receiving a notification indicating attendance state from the host, making it possible to eliminate a trouble of an operation related to the update by the host.

According to the information providing device of note 5, the communication unit of the information providing device transmits, when the confirmation information is received by the communication unit from the client-side device, the confirmation information to the host-side devices other than the host-side device corresponding to the confirmation information among the host-side devices corresponding to the host information specified by the specification unit, so that the confirmation information can be offered to the other host who do not provide service to the client among the hosts of the host-side devices specified by the specification unit, enabling the other host to afterward confirm that service has provided to the client by a host other than he/she.

### [Reference Numerals]

- 1: retrieval system
- 2: network
- 3: first medical facility
- 4: second medical facility
- 10: client-side device
- 11: current location acquisition unit
- 12: client-side communication unit
- 13: touch pad
- 14: display
- 15: speaker
- 16: control unit
- 16a: acquisition unit
- 16b: retrieval condition information generation unit
- 17: data recording unit
- 20: host-side device
- 21: current location acquisition unit
- 22: host-side communication unit
- 23: touch pad
- 24: display
- 25: speaker
- 26: control unit
- 26a: reply information generation unit
- 27: data recording unit
- 30: information providing device
- 31: communication unit
- 32: control unit
- 32a: specification unit
- 32b: notification information generation unit
- 32c: update unit
- 33: data recording unit
- 33a: host DB
- 33b: medical facility DB

## Claims

1. A retrieval system for retrieving a host that provides a service to a client, the retrieval system comprising a client-side device, a plurality of host-side devices, and an information providing system communicably connected to the client-side device and each of the plurality of host-side devices, wherein
the client-side device comprises:
client-side communication unit for communicating with the plurality of host-side devices or the information providing system;
acquisition unit for acquiring generation information for generating retrieval condition information indicating a condition for retrieving the host;
retrieval condition information generation unit for generating the retrieval condition information;
client-side output unit for outputting various pieces of information related to the client-side device; and
a medical care field table storing symptom information indicating a symptom of a patient and information indicating a medical care field in association with each other,
each of the plurality of host-side devices comprises:
host-side communication unit for communicating with the client-side device or the information providing system;
reply information generation unit for generating reply information indicating a reply of whether the service can be provided; and
host-side output unit for outputting various pieces of information related to the each of the host-side devices, and
the information providing system comprises:
communication unit for communicating with the client-side device or the plurality of host-side devices;
host information storage unit for storing the retrieval condition information and host information for specifying the host in association with each other;
specification unit for specifying the host information corresponding to the retrieval condition information from among the host information stored in the host information storage unit when the retrieval condition information is received by the communication unit; and
notification information generation unit for generating notification information for giving a notice of being a host corresponding to the host information specified by the specification unit, and wherein
the retrieval condition information generation unit of the client-side device generates the retrieval condition information based on the generation information when the generation information is acquired by the acquisition unit,
the client-side communication unit of the client-side device transmits the retrieval condition information generated by the retrieval condition information generation unit to the information providing system,
the specification unit of the information providing system specifies the host information corresponding to the retrieval condition information from among the host information stored in the host information storage unit when the retrieval condition information is received by the communication unit,
the communication unit of the information providing system transmits the notification information generated by the notification information generation unit to one or more of the host-side devices corresponding to the host information specified by the specification unit,
the host-side output unit of each of the plurality of host-side devices outputs the notification information when the notification information is received by the host-side communication unit,
the reply information generation unit of each of the plurality of host-side devices generates the reply information on the notification information output by the host-side output unit and that indicates the reply specified by a predetermined method,
the host-side communication unit of each of the plurality of host-side devices transmits the reply information generated by the reply information generation unit to the client-side device or the information providing system,
the client-side output unit of the client-side device outputs the reply information when the reply information is received by the client-side communication unit,
the service is a medical service,
the retrieval condition information includes medical care field information indicating a medical care field of the doctor related to the symptom of the patient and attendance state information indicating an attendance state of the doctor, or location information indicating a location of the doctor related to location of the patient,
the generation information includes medical care field generation information for generating the medical care field information, the medical care field generation information indicating the symptom of the patient, and attendance state generation information for generating the attendance state information, the attendance state generation information indicating the attendance state of the doctor, or location generation information for generating the location information, the location generation information indicating an area in a predetermined distance range from a current location of the patient or a client assistant, and
the retrieval condition information generation unit of the client-side device
specifies, when the medical care field generation information is acquired by the acquisition unit, information indicating the medical care field corresponding to the medical care field generation information from among information indicating the medical care field stored in the medical care field table, and generates the specified information as the medical care field information,
generates, when the attendance state generation information is acquired by the acquisition unit, the attendance state generation information as the attendance state information, and
extracts, when the location generation information is acquired by the acquisition unit, the location information corresponding to coordinates in the area indicated by the location generation information from among the location information acquired by the information providing system or the host-side devices, and generates the extracted information as the location information.

2. A retrieval system for retrieving a host that provides a service to a client, the retrieval system comprising a client-side device, a plurality of host-side devices, and an information providing system communicably connected to the client-side device and each of the plurality of host-side devices, wherein
the client-side device comprises:
client-side communication unit for communicating with the plurality of host-side devices or the information providing system;
acquisition unit for acquiring generation information for generating retrieval condition information indicating a condition for retrieving the host;
retrieval condition information generation unit for generating the retrieval condition information; and
client-side output unit for outputting various pieces of information related to the client-side device,
each of the plurality of host-side devices comprises:
host-side communication unit for communicating with the client-side device or the information providing system;
reply information generation unit for generating reply information indicating a reply of whether the service can be provided; and
host-side output unit for outputting various pieces of information related to the each of the host-side devices, and
the information providing system comprises:
communication unit for communicating with the client-side device or the plurality of host-side devices;
host information storage unit for storing the retrieval condition information and host information for specifying the host in association with each other;
specification unit for specifying host information corresponding to the retrieval condition information from among the host information stored in the host information storage unit when the retrieval condition information is received by the communication unit, and
notification information generation unit for generating notification information for giving a notice of being a host corresponding to the host information specified by the specification unit, and wherein
the retrieval condition information generation unit of the client-side device generates the retrieval condition information based on the generation information when the generation information is acquired by the acquisition unit,
the client-side communication unit of the client-side device transmits the retrieval condition information generated by the retrieval condition information generation unit to the information providing system,
the specification unit of the information providing system specifies the host information corresponding to the retrieval condition information from among the host information stored in the host information storage unit when the retrieval condition information is received by the communication unit,
the communication unit of the information providing system transmits the notification information generated by the notification information generation unit to one or more of the host-side devices corresponding to the host information specified by the specification unit,
the host-side output unit of each of the plurality of host-side devices outputs the notification information when the notification information is received by the host-side communication unit,
the reply information generation unit of each of the plurality of host-side devices generates the reply information on the notification information output by the host-side output unit and that indicates the reply specified by a predetermined method,
the host-side communication unit of each of the plurality of host-side devices transmits the reply information generated by the reply information generation unit to the client-side device or the information providing system,
the client-side output unit of the client-side device outputs the reply information when the reply information is received by the client-side communication unit,
the service is a medical service,
the retrieval condition information includes attendance state information indicating an attendance state of the doctor, or location information indicating a location of the doctor related to a location of the patient,
the generation information includes attendance state generation information for generating the attendance state information, the attendance state generation information indicating the attendance state of the doctor, or location generation information for generating the location information, the location generation information indicating an area in a predetermined distance range from a current location of the patient or a client assistant, and
the retrieval condition information generation unit of the client-side device
generates, when the attendance state generation information is acquired by the acquisition unit, the attendance state generation information as the attendance state information, and
extracts, when the location generation information is acquired by the acquisition unit, the location information corresponding to coordinates in the area indicated by the location generation information from among the location information acquired by the information providing system or the host-side devices, and generates the extracted information as the location information.

3. An information providing system communicably connected to a client-side device and each of a plurality of host-side devices, the information providing system comprising:
communication unit for communicating with the client-side device or the plurality of host-side devices;
host information storage unit for storing retrieval condition information indicating a condition for retrieving a host that provides a service to a client, and host information for specifying the host in association with each other;
specification unit for specifying host information corresponding to the retrieval condition information from among the host information stored in the host information storage unit when the retrieval condition information is received by the communication unit; and
notification information generation unit for generating notification information for giving a notice of being a host corresponding to the host information specified by the specification unit, wherein
the specification unit specifies the host information corresponding to the retrieval condition information from among the host information stored in the host information storage unit when by the communication unit receives the retrieval condition information from the client-side device,
the communication unit transmits the notification information generated by the notification information generation unit to one or more of the host-side devices corresponding to the host information specified by the specification unit,
the service is a medical service, and
the retrieval condition information includes location information indicating a location of the doctor related to a location of the patient.

4. The information providing system according to claim 3 comprising update unit for updating, when doctor current location information indicating a current location of the doctor and medical facility location information indicating a location of a medical facility to which the doctor belongs are acquired by a predetermined method, the attendance state information among the retrieval condition information stored in the host information storage unit based on the doctor current location information and the medical facility location information.

5. The information providing system according to claim 3 or claim 4, wherein
the communication unit of the information providing system transmits, when the communication unit receives confirmation information indicating that the host that provides the service has confirmed from the client-side device, the confirmation information to one or more of the host-side devices other than one of the host-side devices corresponding to the confirmation information from among some of the host-side devices corresponding to the host information specified by the specification unit.

6. A client-side device communicably connected to an information providing device and each of a plurality of host-side devices, the client-side device comprising:
client-side communication unit for communicating with the plurality of host-side devices or the information providing system;
acquisition unit for acquiring generation information for generating retrieval condition information indicating a condition for retrieving a host;
retrieval condition information generation unit for generating the retrieval condition information;
client-side output unit for outputting various pieces of information related to the client-side device; and
a medical care field table storing symptom information indicating a symptom of a patient and information indicating a medical care field in association with each other, wherein
the retrieval condition information generation unit generates the retrieval condition information based on the generation information when the generation information is acquired by the acquisition unit,
the client-side communication unit transmits the retrieval condition information generated by the retrieval condition information generation unit to the information providing system,
the client-side output unit outputs, when the client-side communication unit receives reply information indicating a reply of whether a service can be provided to a client, the reply information,
the service is a medical service,
the retrieval condition information includes medical care field information indicating a medical care field of the doctor related to the symptom of the patient and attendance state information indicating an attendance state of the doctor, or location information indicating a location of the doctor related to location of the patient,
the generation information includes medical care field generation information for generating the medical care field information, the medical care field generation information indicating the symptom of the patient, and attendance state generation information for generating the attendance state information, the attendance state generation information indicating the attendance state of the doctor, or location generation information for generating the location information, the location generation information indicating an area in a predetermined distance range from a current location of the patient or a client assistant, and
the retrieval condition information generation unit
specifies, when the medical care field generation information is acquired by the acquisition unit, information indicating the medical care field corresponding to the medical care field generation information from among information indicating the medical care field stored in the medical care field table, and generates the specified information as the medical care field information, and
extracts, when the location generation information is acquired by the acquisition unit, the location information corresponding to coordinates in the area indicated by the location generation information from among the location information acquired by the information providing system or the host-side devices, and generates the extracted information as the location information.

7. A client-side device communicably connected to an information providing device and each of a plurality of host-side devices, the client-side device comprising:
client-side communication unit for communicating with the plurality of host-side devices or the information providing system;
acquisition unit for acquiring generation information for generating retrieval condition information indicating a condition for retrieving a host;
retrieval condition information generation unit for generating the retrieval condition information;
client-side output unit for outputting various pieces of information related to the client-side device; and
a medical care field table storing symptom information indicating symptom of a patient and information indicating a medical care field in association with each other, wherein
the retrieval condition information generation unit generates the retrieval condition information based on the generation information when the generation information is acquired by the acquisition unit,
the client-side communication unit transmits the retrieval condition information generated by the retrieval condition information generation unit to the information providing system,
the client-side output unit outputs, when the client-side communication unit receives reply information indicating a reply of whether a service can be provided to a client, the reply information,
the service is a medical service,
the retrieval condition information includes attendance state information indicating an attendance state of a doctor, or location information indicating a location of the doctor related to a location of the patient,
the generation information includes attendance state generation information for generating the attendance state information, the attendance state generation information indicating the attendance state of the doctor, or location generation information for generating the location information, the location generation information indicating an area in a predetermined distance range from a current location of the patient or a client assistant, and
the retrieval condition information generation unit of the client-side device
generates, when the attendance state generation information is acquired by the acquisition unit, the attendance state generation information as the attendance state information, and
extracts, when the location generation information is acquired by the acquisition unit, the location information corresponding to coordinates in the area indicated by the location generation information from among the location information acquired by the information providing system or the host-side devices, and generates the extracted information as the location information.

8. An information providing method performed in an information providing system communicably connected to a client-side device and each of a plurality of host-side devices, the information providing method comprising:
a communication step for communication unit communicating with the client-side device or the plurality of host-side devices;
a specification step for specification unit specifying, when retrieval condition information indicating a condition for retrieving a host that provides a service to a client is received by the communication unit, host information corresponding to the received retrieval condition information from among host information for specifying the host stored in host information storage unit with the retrieval condition information in association to each other; and
a notification information generation step for notification information generation unit generating notification information for giving a notice of being a host corresponding to the host information specified in the specification step; wherein
in the specification step, the host information corresponding to the retrieval condition information is specified from among the host information stored in the host information storage unit when the retrieval condition information is received from the client-side device in the communication step,
in the communication step, the notification information generated in the notification information generation step is transmitted to one or more of the host-side devices corresponding to the host information specified in the specification step,
the service is a medical service, and
the retrieval condition information includes location information indicating a location of the doctor related to a location of the patient.

9. An information providing program for being executed by an information providing system communicably connected to a client-side device and each of a plurality of host-side devices, the information providing program causing a computer to function as:
communication unit for communicating with the client-side device or the plurality of host-side devices;
host information storage unit for storing retrieval condition information indicating a condition for retrieving a host that provides a service to a client and host information for specifying the host in association with each other;
specification unit for specifying host information corresponding to the retrieval condition information from among the host information stored in the host information storage unit when the retrieval condition information is received by the communication unit; and
notification information generation unit for generating notification information for giving a notice of being a host corresponding to the host information specified by the specification unit, wherein
the specification unit specifies the host information corresponding to the retrieval condition information from among the host information stored in the host information storage unit when the communication unit receives the retrieval condition information from the client-side device,
the communication unit transmits the notification information generated by the notification information generation unit to one or more of the host-side devices corresponding to the host information specified by the specification unit,
the service is a medical service, and
the retrieval condition information includes location information indicating a location of the doctor related to a location of the patient.

10. A client-side program for being executed by a client-side device communicably connected to an information providing system and each of a plurality of host side devices, the client-side program causing a computer to function as:
client-side communication unit for communicating with the plurality of host-side devices or the information providing system;
acquisition unit for acquiring generation information for generating retrieval condition information indicating a condition for retrieving a host;
retrieval condition information generation unit for generating the retrieval condition information;
client-side output unit for outputting various pieces of information related to the client-side device; and
a medical care field table storing symptom information indicating a symptom of a patient and information indicating a medical care field in association with each other, wherein
the retrieval condition information generation unit generates the retrieval condition information based on the generation information when the generation information is acquired by the acquisition unit,
the client-side communication unit transmits the retrieval condition information generated by the retrieval condition information generation unit to the information providing system,
the client-side output unit outputs, when the client-side communication unit receives reply information indicating a reply of whether a service can be provided to a client, the reply information,
the service is a medical service,
the retrieval condition information includes medical care field information indicating a medical care field of the doctor related to the symptom of the patient, and attendance state information indicating an attendance state of a doctor, or location information indicating a location of the doctor related to location of the patient,
the generation information includes medical care field generation information for generating the medical care field information, the medical care field generation information indicating the symptom of the patient, and attendance state generation information for generating the attendance state information, the attendance state generation information indicating the attendance state of the doctor, or location generation information for generating the location information, the location generation information indicating an area in a predetermined distance range from a current location of the patient or a client assistant, and
the retrieval condition information generation unit
specifies, when the medical care field generation information is acquired by the acquisition unit, information indicating the medical care field corresponding to the medical care field generation information from among information indicating the medical care field stored in the medical care field table, and generates the specified information as the medical care field information,
generates, when the attendance state generation information is acquired by the acquisition unit, the attendance state generation information as the attendance state information, and
extracts, when the location generation information is acquired by the acquisition unit, the location information corresponding to coordinates in the area indicated by the location generation information from among the location information acquired by the information providing system or the host-side devices, and generates the extracted information as the location information.

11. A client-side program for being executed by a client-side device communicably connected to an information providing system and each of a plurality of host side devices, the client-side program causing a computer to function as:
client-side communication unit for communicating with the plurality of host-side devices or the information providing system;
acquisition unit for acquiring generation information for generating retrieval condition information indicating a condition for retrieving a host;
retrieval condition information generation unit for generating the retrieval condition information;
client-side output unit for outputting various pieces of information related to the client-side device; and
a medical care field table storing symptom information indicating symptom of a patient and information indicating a medical care field in association with each other, wherein
the retrieval condition information generation unit generates the retrieval condition information based on the generation information when the generation information is acquired by the acquisition unit,
the client-side communication unit transmits the retrieval condition information generated by the retrieval condition information generation unit to the information providing system,
the client-side output unit outputs, when the client-side communication unit receives reply information indicating a reply of whether a service can be provided to a client, the reply information,
the service is a medical service,
the retrieval condition information includes attendance state information indicating an attendance state of a doctor, or location information indicating a location of the doctor related to a location of the patient,
the generation information includes attendance state generation information for generating the attendance state information, the attendance state generation information indicating the attendance state of the doctor, or location generation information for generating the location information, the location generation information indicating an area in a predetermined distance range from a current location of the patient or a client assistant, and
the retrieval condition information generation unit of the client-side device
generates, when the attendance state generation information is acquired by the acquisition unit, the attendance state generation information as the attendance state information, and
extracts, when the location generation information is acquired by the acquisition unit, the location information corresponding to coordinates in the area indicated by the location generation information from among the location information acquired by the information providing system or the host-side devices, and generates the extracted information as the location information.
